(19) Europäisches Patentamt
European Patent Office
Office européen des brevets

(11) Numéro de publication: **0 354 108 B1**

(12) # FASCICULE DE BREVET EUROPEEN

(45) Date de publication de fascicule du brevet: **09.03.94**

(51) Int. Cl.[5]: **C07K 7/06**, C07F 9/38, C07F 9/40, C07K 5/02

(21) Numéro de dépôt: **89402164.1**

(22) Date de dépôt: **28.07.89**

(54) **Amino-acides et peptides présentant un résidu tyrosine modifiée, leur préparation et leur application comme médicaments.**

(30) Priorité: **01.08.88 FR 8810391**

(43) Date de publication de la demande:
**07.02.90 Bulletin 90/06**

(45) Mention de la délivrance du brevet:
**09.03.94 Bulletin 94/10**

(84) Etats contractants désignés:
**AT BE CH DE ES FR GB GR IT LI LU NL SE**

(56) Documents cités:
**EP-A- 0 226 217**
**EP-A- 0 268 297**
**EP-A- 0 313 002**
**FR-A- 2 606 018**
**US-A- 4 657 899**

**THE JOURNAL OF ORGANIC CHEMISTRY, vol. 53, no. 15, 22 juillet 1988, pages 3621-3624, American Chemical Society; I. MARSEIGNE et al.: "Synthesis of new amino acids mimicking sulfated and phosphorylated tyrosine residues"**

(73) Titulaire: **INSTITUT NATIONAL DE LA SANTE ET DE LA RECHERCHE MEDICALE (INSERM)**
**101, rue de Tolbiac**
**F-75654 Paris Cédex 13(FR)**

(72) Inventeur: **Roques, Bernard Pierre**
**12 rue Eugène Delacroix**
**F-94410 Saint Maurice(FR)**
Inventeur: **Marseigne, Isabelle**
**314 rue Saint Jacques**
**F-75005 Paris(FR)**
Inventeur: **Charpentier, Bruno**
**Le Clos Marella**
**6 Allée des Troenes**
**F-94240 L'Hay Les Roses(FR)**

(74) Mandataire: **Phélip, Bruno et al**
**c/o Cabinet Harlé & Phélip**
**21, rue de La Rochefoucauld**
**F-75009 Paris (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

**NEUROLOGY NEUROBIOLOGY, vol. 46, 1988, pages 231-234, Alan R. Liss Inc.; J.T. DRUMMOND et al.: "Aryl-spaced APH analogs are NMDA receptor antagonists and protect against NMDA-induced convulsions and death in the mouse"**

## Description

La présente invention concerne de nouveaux peptides. Ces nouveaux peptides, comportent, dans leur séquence, un résidu tyrosine modifiée, avec, en particulier, un enchaînement spécifique d'amino-acides.

L'invention concerne également la préparation de ces nouveaux composés, ainsi que leur application comme médicaments, notamment dans le traitement des maladies nerveuses d'origine centrale.

La présence d'un résidu tyrosine sulfatée a été mise en évidence dans de nombreux peptides (fibrinopeptide B [BETTELHEIM, F.R., J. Am. Chem. Soc., 76, 2838 (1954)], gastrine [GREGORY, H. Nature, 204, 931 (1964)], cholécystokinine [MUTT, V., Eur. J. Biochem., 6, 156, (1968)], et plus récemment, dans plusieurs protéines sécrétrices (Immunoglobine G [BAEUERLE. P.A., EMBO J., 3, 2209 (1984)], fibronectine [LIU, M.C., Proc. Natl. Acad. Sci. USA, 82, 34, (1985)].

La sulfatation du groupe -OH phénolique de l'aminoacide tyrosine est vraisemblablement un évènement post-traductionnal qui se produit au niveau des résidus tyrosine de la procholécystokinine [VARGAS, F., Ann. NY Acad. Sci., 448, 110 (1985)]. Ce processus pourrait également intervenir dans le cas des précurseurs des enképhalines, préproenképhaline [UNSWORTH, C.D., Nature, 295, 519-522, (1982)]. Enfin, la sulfatation de l'amino-acide tyrosine seul pourrait également exister dans les milieux biologiques [RONDOUIN, G., Neuropept., 1, 23-28 (1980)].

L'intérêt de la tyrosine sulfatée dans les processus biologiques est donc manifeste. Toutefois, l'intérêt est limité par le fait que la liaison -O-$SO_3H$ est facilement hydrolysable [BETTELHEIM, F.R., J. Am. Chem. Soc., 76, 2838-2839. (1954)].

Une autre modification post-traductionnelle des protéines, qui s'avère être un processus important dans la régulation cellulaire est la phosphorylation des résidus tyrosines [HUNTER, T., Cell, 22, 647-648 (1980) ; USHIRO, H., J. Biol. Chem., 255, 8363-8365 (1980)]. La phosphorylation des protéines, notamment des protéines membranaires (récepteurs, transducteurs, etc..), s'effectue en effet tout particulièrement au niveau de la tyrosine des dérivés peptidiques, sous l'action d'enzymes dénommées tyrosine-kinases, dont certaines correspondraient à des protéines d'expression des oncogènes [HUNTER, T., Ann. Rev. Biochem., 54, (1985), 897-930].

Par ailleurs, de nombreux agonistes ou antagonistes des récepteurs aux amino-acides excitateurs présentent des structures dérivées des $\alpha$-amino-acides, caractérisées par une chaîne latérale aliphatique terminée par une fonction acide sulfonique ou phosphonique [WATKINS, J.C., TINS, 10(7), (1987), 265-272].

La présente invention concerne de nouveaux peptides comportant un résidu tyrosine O-sulfatée ou O-phosphorylé pouvant être modifiés et comportant un enchaînement spécifique d'amino-acides, ce par quoi ils sont également susceptibles, de par leur plus grande stabilité chimique et enzymatique, d'avoir une plus longue durée d'action lorsqu'ils sont administrés dans un organisme vivant.

Parmi les nouveaux peptides selon l'invention, sont plus particulièrement intéressants les peptides présentant des propriétés cholécystokininergiques.

Ainsi la présente invention concerne tout particulièrement, mais non exclusivement, de nouveaux peptides présentant des propriétés cholécystokininergiques, leur préparation et les compositions thérapeutiques qui les contiennent.

On sait qu'il existe au moins deux types de récepteurs cholécystokininergiques, de poids moléculaires différents [SAKAMOTO et al., J. Biol. Chem. 258, 12707 (1983) ; SAKAMOTO et al., Biochem. Biophys. Res. Comm., 124, 497 (1984)], capables de lier l'octapeptide sulfaté suivant (connu sous le nom de $CCK_8$) :

Asp-Tyr($SO_3H$)-Met-Gly-Trp-Met-Asp-Phe-$NH_2$.

On distingue ainsi les récepteurs du système nerveux central et les récepteurs du système nerveux périphérique. Au niveau du système nerveux central, la $CCK_8$ joue un rôle de neurotransmetteur [GOLTERMAN et al., J. Biol. Chem. 255, 6181 (1908)]. De plus, elle est colocalisée avec la dopamine dans certains neurones de la voie mésolimbique [HOKFELT et al., Nature, 285, 476 (1980)]; elle en antagonise les effets dans le système mésolimbique et facilite la neurotransmission dopaminergique dans le striatum [FUXE et al., Eur. J. Pharmacol., 67, 329 (1980)]. Les récepteurs périphériques, eux, sont impliqués dans la contraction des muscles lisses au niveau intestinal [HUTCHINSON et al., Eur. J. Pharmacol., 69, 87 (1981) ; CHANG et al., Neuroscience Lett., 46, 71 (1984)] et dans la libération d'amylase pancréatique [JENSEN et al., J. Biol. Chem., 257, 5554 (1982)].

La $CCK_8$, ainsi que d'autres produits structurellement apparentés sont connus par la demande de brevet allemand 3.138.233 pour avoir une action neuropsychrotrope . Par ailleurs , ce peptide possède des propriétés analgésiques ( Barbaz et al., Neuropharmacology, 25, 823 ( 1986) ) ; il facilite les processus de

mémorisation ( Katsuura et Itoh, Peptides, 87, 105 ( 1986)) ; il a des propriétés anorexigènes ( Gibbs et al. J. Comp. Physiol. Psychol. 84, 488 ( 1973)), et également , il accèlère la motilité intestinale ( Mutt. Gastrointestinal hormones, Jersey Glass, G.B. Ed. Raven Press, New York, pages 169-221 ( 1980)) .

On connaît , par le brevet allemand précité , des analogues de la $CCK_8$ qui possèdent , dans la séquence peptidique , la tyrosine O-sulfatée comme amino-acide , et qui ne présentent , dans leur séquence , que des amino acides naturels . Toutefois , l'intérêt pratique de tels produits est limité du fait que la liaison $-O-SO_3H$ est facilement hydrolysable comme l'a rapporté BETTELHEIM F.R., J. Am. Soc. 76, 2838 ( 1954) . De même , ces produits sont rapidement hydrolysés au niveau des liaisons peptidiques par différentes classes de protéases ( Durieux et al. Neuropeptides , 7 . 1-9 ( 1986) ).

Un autre document , un article paru dans The Journal of Organic Chemistry ( Marseigne et al., 53, N°15 , 3621-3624, (1988)) décrit aussi la synthèse d'un dérivé de la $CCK_8$ , ledit dérivé ayant un phényl substitué en para par un groupement $CH_2SO_3H$ à la place de la tyrosine . Ce document décrit en outre la synthèse de nouveaux acides aminés .

Deux demandes européennes EP-226.217 et EP-268.297 concernent des peptides de 6 à 9 acides aminés contenant un résidu tyrosine dont le groupement hydroxyle est substitué par un groupement $-OSO_3H$ .

Il était donc intéressant de rechercher des produits analogues de la CCK8 mais présentant une plus grande stabilité , tout en conservant ou en améliorant les propriétés de la $CCK_8$ . Il est particulièrement important d'observer que certaines modifications de la séquence des peptides cholecystokininergiques , qui sont effectuées selon l'invention, conduisent à des peptides très affins et sélectifs envers les récepteurs centraux à CCK ( type B ), par rapport aux récepteurs périphériques à CCK ( type A) , les rendant ainsi utilisables comme outils thérapeutiques spécifiques du système nerveux central .

La présente invention a donc d'abord pour objet des composés chimiques représentés par la formule(I):

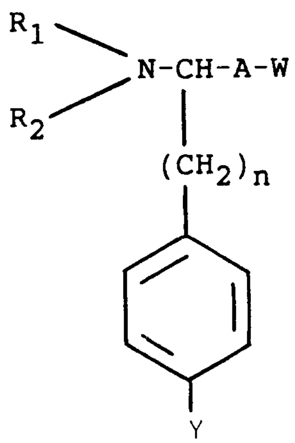

( I )

dans laquelle :

R1 représente un atome d'hydrogène ;

R2 représente un radical protecteur de fonction amine du type acyle ou du type uréthanne ;

A représente un carbonyle;

Y représente un reste choisi parmi ceux de formule :

$OSO_2OR_3$, et

$(CH_2)_m-SO_2OR_3$

où :

- * $R_3$ représente un atome d'hydrogène ou un métal alcalin,
- * m est I , et
- * n est O ou I.

Le radical protecteur de fonction amine du type acyle , entrant dans la définition de $R_2$, est , par exemple , un radical formyle, acétyle, chloracétyle , trifluoracétyle , propionyle, butyryle, isobutyryle , gamma-chlorobutyryle, oxalyle, succinyle, glutamyle , pyroglutamyle, phtalyle, p-toluènesulfonyle .

Quant au radical protecteur de fonction amine du type uréthanne , entrant dans la définition de $R_2$ , c'est , par exemple , un radical tert-butyloxycarbonyle, isopropyloxycarbonyle, éthoxycarbonyle, allyloxycarbonyle, benzyloxycarbonyle , mono- ou polyhalobenzyloxycarbonyle.

W représente :

- B - D - Trp - E - Asp - PheNHQ,

où

* B et E , représentent un reste norleucine, ou de ses dérivés N-méthylés correspondants ,
* D représente un reste glycine ;
* Q représente un hydrogène ,

lesdits composés se présentant sous les formes D, L ou DL, ainsi que leurs mélanges et leurs sels pharmaceutiquement acceptables .

Selon la définition de l'invention , par " séquence d'amino-acides naturels " , on entend l'une quelconque des combinaisons , dans un ordre quelconque, des amino-acides tels que décrits dans l'ouvrage " Biochimie " de Lehninger, page 67, Ed. Flammarion-Médecine-Science .

La présente invention a également pour objet des procédés de préparation des composés représentés par la formule ( I ) telle que définie ci-dessus .

Les amino-acides comportant un résidu tyrosine modifiée et inclus dans les composés objets de la présente invention peuvent être obtenus par le procédé suivant .

Dans le cas où Y représente un radical de formule $(CH_2)_m$-$SO_2OR_3$ , ce procédé est caractérisé par le fait que :

- on fait réagir un sulfite alcalin (sulfite de sodium) sur un composé de formule générale (VI) :

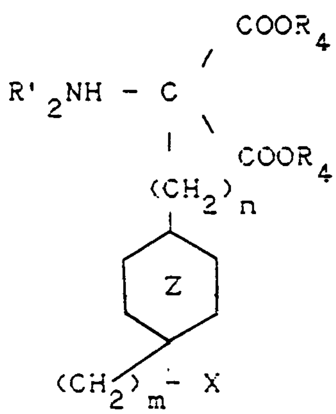

(VI)

dans laquelle :

- $R'_2$ représente un groupe protecteur de la fonction amine, tel que défini ci-dessus pour $R_2$,
- $R_4$ représente un radical alkyle ;
- X représente halogène, tel que chlore ;
- m, n étant tels que définis ci-dessus ;

et Z étant un noyau phénile

- puis on hydrolyse et on décarboxyle le produit intermédiaire pour obtenir un composé de formule générale (A)

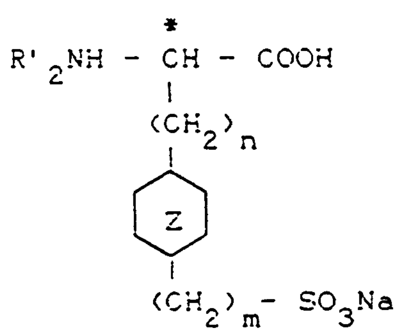

(A)

- puis on isole le composé obtenu et on le transforme, si on le désire, en un autre composé de formule générale (B), dans laquelle Y représente un radical de formule $(CH_2)_mSO_2OR_3$, par toute méthode connue en soi, et on transforme éventuellement le composé obtenu en un sel pharmaceutiquement acceptable.

L'action du sulfite de sodium sur le produit de formule générale (VI) s'effectue généralement par chauffage à une température comprise entre 100 et 120 °C, en présence d'une base minérale aqueuse, telle que la soude ou la potasse. L'hydrolyse et la décarboxylation subséquentes sont effectuées par chauffage à une température comprise entre 100 et 120 °C, en présence d'un acide minéral fort, tel que l'acide chlorhydrique aqueux.

La transformation du produit de formule générale (A) en un autre produit de formule générale (B) peut s'effectuer par toute méthode connue de l'homme du métier pour transformer, par example, un acide carboxylique ou sulfonique en ester, un ester en alcool, en éther ou en amine, ou pour transformer un amide en amine, alkyler ou acyler une amine ou débloquer une fonction amine.

Les produits de formule (VI) peuvent être obtenus par action du chlorure de thionyle sur un alcool de formule générale (VII) :

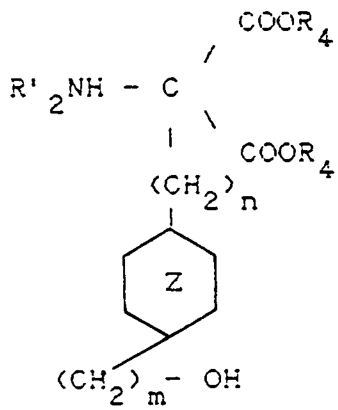

(VII)

dans laquelle les symboles sont définis comme précédemment, en opérant sans solvant ou dans un solvant organique, tel que le chlorure de méthylène, à la température de reflux du mélange réactionnel.

Les produits de formule générale (VII) peuvent être obtenus par action du nitrite de sodium sur un produit de formule générale (VIII) :

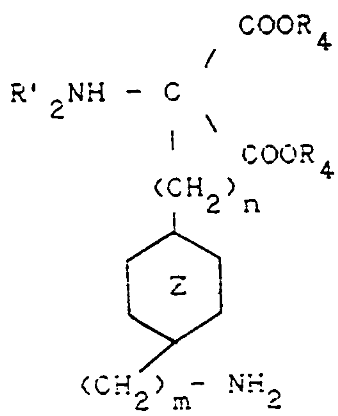

(VIII)

dans laquelle les symboles sont définis comme précédemment, suivie d'une hydrolyse du composé diazoïque formé intermédiairement, selon les conditions habituelles connues de l'homme du métier.

Les produits de formule générale (VIII) peuvent être obtenus par réduction d'un produit de formule générale (IX) :

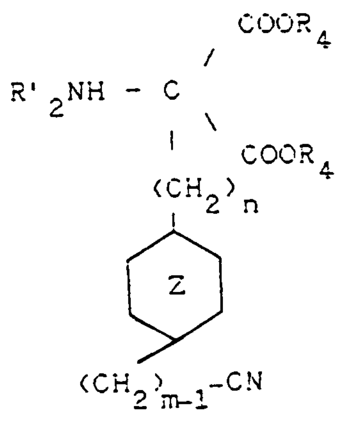

(IX)

dans laquelle les symboles sont définis comme précédemment, en opérant par toute méthode connue, par exemple par réduction au moyen d'hydrogène en présence de catalyseur, comme le palladium sur noir de carbone, dans un alcool, tel que le méthanol ou l'éthanol.

Les produits de formule générale (IX) peuvent être préparés par action d'un produit de formule générale (X) :

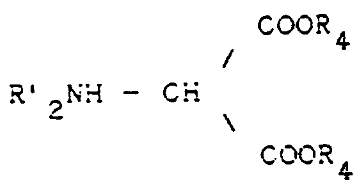

(X)

dans laquelle $R'_2$ et $R_4$ ont les définitions données ci-avant sur un produit de formule générale (XI) :

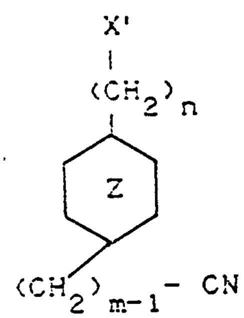

(XI)

dans laquelle X' représente un atome d'halogène, et les autres symboles sont définis comme précédemment, en opérant selon les conditions habituelles, en présence d'une base organique, telle qu'un éthylate alcalin.

Les produits de formule générale (I) dans lequel

- -A représente -CO- : et
- -W représente : -B-D-Trp-E-Asp-Phe-NHQ

B, D, E et Q étant tels que définis ci-dessus, peuvent être préparés de la façon suivante :

- on réalise le couplage d'un peptide de formule générale (XIII) :

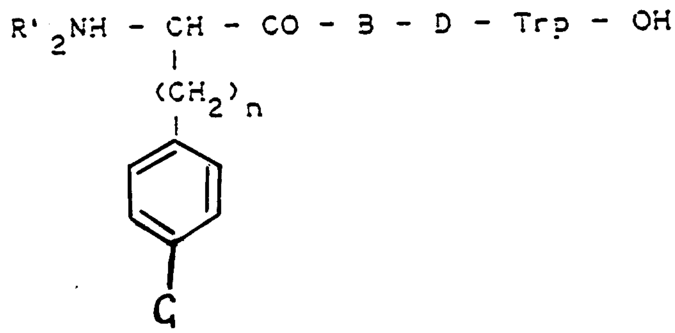

(XIII)

ou un dérivé activé de ce peptide

où :

- Z représente un cycle phényle
- $R'_2$ représente un radical protecteur d'amine tel que défini ci-dessus pour $R_2$ ;
- G représente OH, ou un radical de formule $-(CH_2)_m-SO_2OR_3$, tel que définie en relation avec la formule (I) ; et
- les autres symboles sont tels que définis ci-dessus en relation avec la formule (I) ;

avec le tripeptide de formule (XIV) :

$H\text{-}E\text{-}Asp\text{-}PheNH_2$ (XIV)

où E est tel que défini ci-dessus, ce tripeptide étant éventuellement protégé,

- puis on élimine le (ou les) groupement(s) protecteur(s), et on isole le produit obtenu à l'état libre ou à l'état de sel, et on le transforme éventuellement en un autre produit tel que défini ci-dessus, par toute méthode connue en soi, et on transforme, si on le désire, le produit final en sel pharmaceutiquement acceptable ou en produit à l'état libre selon le cas.

Le couplage des produits de formule générale (XIII) et (XIV) s'effectue par toute méthode connue de l'homme du métier pour condenser un peptide sur un autre peptide.

Il est particulièrement avantageux d'utiliser le peptide de formule générale (XIII) sous une forme activée. Comme forme activée, on peut citer le produit de réaction du composé de formule générale (XIII) avec l'hydroxybenzotriazole, la N-hydroxysuccinimide, le p-nitrophénol ou le tri- ou penta-chlorophénol, préparé en présence d'un agent de condensation.

Dans la pratique, il est particulièrement avantageux d'utiliser, comme forme activée, le produit de réaction avec le N-hydroxysuccinimide dans un solvant organique, tel qu'un éther, comme le tétrahydrofuranne, un solvant chloré, comme le chlorure de méthylène ou le chloroforme, un amide, comme le diméthylformamide, ou un mélange de ces solvants, en présence de dicyclohexylcarbodiimide, à une température voisine de 0 ° C.

Le tripeptide de formule (XIV) peut être obtenu selon la méthode décrite par RUIZ-GAYO et al., Peptides, 6, 415 (1985).

Le produit de formule générale (XIII) peut être obtenu par couplage du peptide de formule (XV) :

H-B-D-Trp-OH (XV)

où B et D sont tels que définis ci-dessus, sur un amino-acide de formule générale (XVI) :

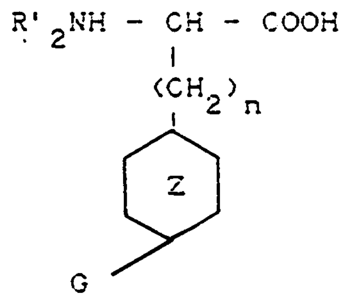

(XVI)

dans laquelle :

- Z représente un cycle phényle
- G représente OH, ou un radical de formule $-(CH_2)_m-SO_2OR_3$, telle que définie en relation avec la formule (I) ;
- les symboles $R'_2$ , n et Z sont tels que définis précédemment, en relation avec la formule (I) et (A).

Le couplage s'effectue par toute méthode connue en soi, en chimie peptidique, notamment par la méthode dont les conditions sont indiquées précédemment, pour coupler ensemble les produits des formules générales (XIII) et (XIV).

Le produit de formule générale (XV) peut être préparé selon la méthode décrite par RUIZ-GAYO et al., Peptides, 6, 415 (1985).

Les produits de formule générale (I) dans lesquels l'enchaînement -A-W représente

-CO-B-D-Trp-E-Asp-Phe-NHQ

(B, D, E et Q étant tels que définis ci-dessus)
peuvent aussi être préparés par couplage d'un peptide de formule générale :

$H-B-D-Trp-E-Asp(OR_5)-Phe-NH-Q$ (XVII)

où :

- B, D, E et Q sont tels que définis ci-dessus: et
- $R_5$ représente un radical protecteur d'acide carboxylique, et, plus particulièrement, un groupe tert.-butyle ou benzyle.

sur un composé de la formule (XVI), telle que définie ci-dessus.

Le couplage s'effectue par toute méthode connue de l'homme du métier pour condenser un aminoacide sur un peptide, notamment selon les procédés indiqués précédemment.

Le produit de formule générale (XVII) est obtenu après déprotection de la fonction amine terminale de l'hexapeptide :

$R''_2-B-D-Trp-E-Asp-Phe-NHQ$ (XVIII)

(où $R''_2$ est défini comme pour $R_2$ et $R'_2$ ),
obtenu par condensation du dipeptide :

$R''_2-B-D-OH$ (XIX)

sur le tétrapeptide de formule :

$H-Trp-E-Asp(OR_5)-Phe-NHQ$ (XX)

formules dans lesquelles $R''_2$, B, D, E, Q et $R_5$ sont tels que définis précédemment.

Il est particulièrement avantageux d'utiliser cette méthode de préparation dans le cas où le résidu B est un reste diamino-1,1 alkyle, diamino-1,1 thiométhylalkyle, diamino-1,1 mercaptoalkyle, ou diamino-1,1 hydroxyalkyle, et le résidu D est un reste acide malonique. Dans tous ces cas, le dipeptide $R''_2$-B-D-OH, présentant alors une liaison rétro-inverso est synthétisé à partir d'un amino-acide $R''_2$-F-OH, -F- étant tel que cet amino-acide soit un précurseur du résidu B tel qu'il vient d'être défini correspondant au reste B

selon une transposition de Curtius.

La synthèse du dipeptide rétro-inverso s'effectue selon la méthode générale décrite par Chorev et Goodman, Int. J. Peptide Protein Research, 21, 258 (1983). L'amino-acide $R''_2$-F-OH est activé sous forme d'azoture d'acyle au niveau de la fonction carboxylique, puis, par chauffage, il se transpose en dérivé isocyanate, sur lequel on condense l'acide malonique pour conduire au dipeptide retro-inverso $R''_2$-B-D-OH (XIX). Il est particulièrement avantageux de protéger la fonction amine par un groupe benzyloxycarbonyle.

Le tétrapeptide de formule générale (XX) est obtenu selon une méthode générale de synthèse peptidique mettant en jeu un procédé pas à pas, à partir du composé H-Phe-NHQ, comme cela a été décrit par Bodanszky et al. J. Med. Chem. 21, 1030 (1978).

Dans le cas où le groupe Y correspond à la formule $-OSO_2OR_3$, la fonction hémiester sulfurique est obtenue par sulfatation de la fonction phénol de la tyrosine d'un peptide, à l'aide du complexe anhydride sulfurique-pyridine.

Comme le remarquera facilement l'homme du métier, il est nécessaire, pour mettre en oeuvre le procédé selon l'invention, d'effectuer, à divers stades de la synthèse, des réactions sur des produits dont les fonctions acides ou amines auront dû au préalable être protégées. Dans ce cas, le radical protecteur sera éliminé au moment le plus opportun de la synthèse, notamment avant couplage des acides aminés ou des peptides entre eux. A titre non limitatif, on pourra, par exemple, protéger les fonctions de la manière suivante :

- Pour les fonctions amine, on pourra utiliser les radicaux protecteurs énumérés pour la définition du symbole $R'_2$ précédent. Lorsque le blocage s'effectue sur un produit intermédiaire et est donc destiné à être éliminé par la suite, il est avantageux d'utiliser le radical tert-butyloxycarbonyle ; celui-ci pourra ensuite être éliminé dans des conditions relativement douces, par example en milieu acide au moyen d'acide trifluoroacétique dilué ou non, dans le chlorure de méthylène, ou au moyen d'une solution d'acide chlorhydrique gazeux dans un solvant anhydre, tel que le dioxanne ou l'acide acétique ; on isole alors souvent le peptide ou l'acide aminé sous forme de trifluoroacétate ou d'acétate. La base pourra être libérée au moment que l'on désire, à l'aide d'une base plus forte telle que la triéthylamine ou la N,N'-diisopropyléthylamine.
- Pour les fonctions acides, on pourra, par exemple, utiliser les radicaux ester habituellement employés en chimie peptidique et facilement éliminables par la suite.

Il est particulièrement avantageux de bloquer les fonctions acides sous forme d'esters méthyliques ou éthyliques qui seront ensuite éliminés par saponification au moyen d'une solution aqueuse d'un hydroxyde alcalin, tel que la soude.

Lorsqu'en plus de la fonction acide C-terminale, il existe une autre fonction acide portée par la chaîne latérale d'un amino-acide, tel que l'acide aspartique, cette fonction acide pourra être avantageusement bloquée par un autre type de radical afin de débloquer ensuite les fonctions acides sélectivement. Dans le cas de l'acide aspartique par exemple, on pourra employer un radical benzyloxy que l'on pourra éliminer ensuite par hydrogénolyse en présence d'un catalyseur, tel que le palladium sur charbon actif.

Les formes diastéréoisomères des peptides résultant de l'introduction, dans leur séquence, du résidu tyrosine modifiée, dans lequel Y correspond à la formule $-(CH_2)_m-SO_2OR_3$, peuvent éventuellement être séparées selon les méthodes habituelles comme la chromatographie.

Les nouveaux produits de formule générale (I), ainsi que les intermédiaires de synthèse; peuvent être purifiés, le cas échéant, par les méthodes habituelles, telles que le cristallisation, la chromatographie ou le formation de sels.

Lorsque les produits de formule générale (I) possèdent, dans leur molécule, une fonction amine libre, ils peuvent être convertis en sels d'addition avec les acides, par action d'un acide, en opérant dans un solvant organique, tel qu'un alcool, une cétone, un éther ou un solvant chloré. Le sel précipite, éventuellement après concentration de sa solution ; il est séparé par filtration ou décantation.

Comme sels d'addition avec les acides, on peut citer les sels d'acides minéraux, tels que chlorhydrates, sulfates ou phosphates, et les sels d'acides organiques, tels qu'acétates, propionates, succinates, benzoates, fumarates, maléates, méthanesulfonates, iséthionates, théophillineacétates, salicylates, phénolphtalinates, méthylène bis-$\beta$-oxynaphtoates ou des dérivés de substitution de ces composés.

Lorsque les produits de formule générale (I) renferment dans leur molécule une fonction acide salifiable, il est parfois avantageux de les isoler sous forme de sel de sodium ou de potassium. Les acides libres peuvent ensuite être, si on le désire, libérés de leurs sels selon les techniques habituelles et être, si on le désire retransformés en un autre sel avec une base.

Comme sels avec les bases, on peut citer par exemple, les sels de sodium, de potassium ou les sels d'addition avec les bases azotées tels que les sels d'éthanolamine ou de lysine.

Les nouveaux composés selon l'invention de formule générale (I) présentent d'intéressantes propriétés pharmacologiques les rendant utiles dans le traitement des maladies nerveuses d'origine centrale, en particulier, dans le cas où ces composés présentent une sélectivité pour les récepteurs de type centraux ; les composés de formule (I) présentent également un intérêt pour leurs propriétés analgésiques, anorexigènes et stimulantes de la motilité intestinale.

Les amino-acides et leurs dérivés se sont en effet montrés actifs in vitro dans le test d'affinité vis-à-vis des récepteurs aux amino-acides excitateurs. Ces propriétés sont mises en évidence par la mesure du pouvoir inhibiteur ($K_I$) qu'exercent les produits sur la liaison de l'acide D-amino-2 phosphono-5 pentanoïque tritié, que l'on note $[^3H]$D-$AP_5$, sur des membranes de cortex de rats, selon la technique décrite par OLVERMAN et al., Nature 307, 460-462 (1984) et Eur. J. Pharmacol., 131, 161-162 (1986).

Les produits selon l'invention présentent une toxicité faible. Leur $DL_{50}$ est généralement comprise entre 50 et 100 mg/kg par voie intraveineuse et entre 100 et 150 mg/kg par voie sous-cutanée, chez la souris.

Les peptides selon l'invention se sont également montrés actifs in vitro dans les tests d'affinité vis-à-vis des récepteurs du système nerveux central et périphérique (pancréas, muscles lisses intestinaux). Ces propriétés sont mises en évidence, d'une part, dans des tests de liaison sur membranes de cerveau de souris, et, d'autre, part dans des tests pharmacologiques de libération d'amylase pancréatique de cobaye et de contraction d'iléon de cobaye.

La présente invention concerne également les médicaments constitués par un produit de formule générale (I), sous forme libre ou sous forme de sel d'addition avec un acide ou une base pharmaceutiquement acceptable, à l'état pur ou sous forme d'une composition dans laquelle il est associé à tout autre produit pharmaceutiquement compatible, pouvant être inerte ou physiologiquement actif. Les médicaments selon l'invention peuvent être utilisés par voie orale, parentérale, rectale ou sous forme de patches.

Comme compositions solides pour administration orale, peuvent être utilisés des comprimés, pilules, poudres (notamment dans des capsules de gélatine ou des cachets) ou granulés. Dans ces compositions, le produit actif selon l'invention est mélangé à un ou plusieurs diluants inertes, tels qu'amidon, cellulose, saccharose, lactose ou silice. Ces compositions peuvent également comprendre des substances autres que les diluants, par exemple un ou plusieurs lubrifiants, tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes, tels que l'eau, l'éthanol, le glycérol, les huiles végétales ou l'huile de paraffine. Ces compositions peuvent également comprendre des substances autres que les diluants, par exemple des produits mouillants, édulcorants, épaississants, aromatisants ou stabilisants.

Les compositions stériles pour administration parentérale peuvent être, de préférence, des solutions aqueuses on non aqueuses, des suspensions ou des émulsions. Comme solvant ou véhicule, on peut employer l'eau, le propylèneglycol, un polyéthylèneglycol, des huiles végétales, en particulier, l'huile d'olive, des esters organiques injectables, par exemple, l'oléate d'éthyle ou autres solvants organiques convenables. Ces compositions peuvent également contenir des adjuvants, en particulier, des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Les compositions pour administration rectale sont les suppositoires ou les capsules rectales, qui contiennent, outre le produit actif, des excipients, tels que le beurre de cacao, des glycérides semi-synthétiques ou des polyéthylèneglycols.

En thérapeutique humaine, les amino-acides et leurs dérivés, ainsi que les peptides selon l'invention, sont particulièrement utiles dans le traitement et la prévention des affections liées aux destructions neuronales produites par les ischémies avec anoxie, les dommages causés au tissu cérébral par les crises d'hypoglycémie en particulier chez l'adulte âgé, les crises épileptiques et plus généralement les maladies dégénératrices liées au vieillissement (maladie d'Alzheimer) ou génétique (chorée de Huntington) ainsi que pour leur action facilitante sur les processus de mémorisation ; et les peptides selon l'invention sont particulièrement utiles dans le traitement des affections du système nerveux central dans lesquelles se produit un dysfonctionnement des voies dopaminergiques par exemple dans la maladie de Parkinson, les chorées, la schizophrénie, les dyskinésies tardives ou les épisodes maniaco-dépressifs.

Ainsi, les composés de formule (I) présentent des propriétés antipsychotiques, les rendant utiles comme neuroleptiques ; un effet facilitant les processus de mémorisation, les rendant utiles chez des sujets âgés pour pallier le vieillissement des cellules nerveuses ; des propriétés analgésiques ; ils sont utiles comme anorexigènes, et ils ont des effets stimulant la motilité intestinale, les rendant utiles pour activer le

transit intestinal.

Les doses dépendent de l'effet recherché et de la durée du traitement ; elles sont généralement comprises entre 2 et 10 mg par jour par voie parentérale pour un adulte en une ou plusieurs prises.

D'une façon générale, le médecin déterminera la posologie qu'il estime la plus appropriée en fonction de l'âge, du poids et de tous les autres facteurs propres au sujet à traiter.

Pour mieux illustrer l'objet de l'invention, on va en décrire maintenant plusieurs exemples de réalisation.

Exemple 1 : N-acétyl sulfonatométhyl-4 phénylalanine monosodique

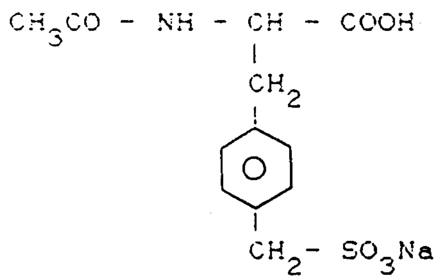

Etape 1 : Préparation de l'acétamido-2 (cyano-4 benzyl)-2 malonate d'éthyle

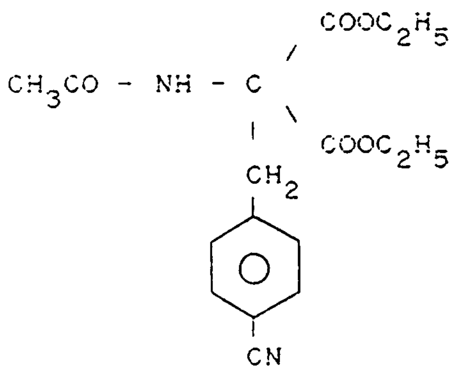

A 25 $cm^3$ d'éthanol anhydre, on ajoute 0,26 g de sodium. Lorsque tout le sodium a disparu, on ajoute à la solution 2,17 g d'acétamido malonate d'éthyle et on chauffe le mélange pendant 15 minutes à une température voisine de 110 °C. A la solution légèrement trouble obtenue, on ajoute, en 15 minutes, 1,96 g d'$\alpha$-bromo p-tolunitrile et on chauffe le mélange réactionnel pendant 17 heures à une température voisine de 110 °C. Après refroidissement, on ajoute 50 $cm^3$ d'eau distillée tout en maintenant l'agitation. Le précipité qui se forme est séparé par filtration et lavé avec deux fois 10 $cm^3$ d'eau distillée, puis séché sous pression réduite (1 mm de mercure ; 0, 13 kPa) à 40 °C. On obtient ainsi 2,89 g d'acétamido-2 (cyano-4 benzyl)-2 malonate d'éthyle sous forme de cristaux blancs.

Rf : 0,73 [chromatographie sur couche mince de gel de silice ; solvant : chloroforme-méthanol (90-10 en volumes)].

Etape 2 : Préparation du chlorhydrate d'acétamido-2 (aminométhyl-4 benzyl)-2 malonate d'éthyle

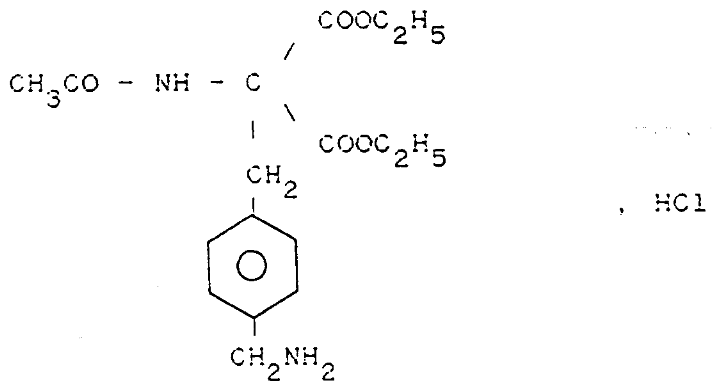

A une suspension de 200 mg de catalyseur au palladium sur noir de carbone dans 4 $cm^3$ d'éthanol, on fait passer un courant d'hydrogène pendant 1 heure jusqu'à saturation, puis on ajoute une solution de 996 mg d'acétamido-2 (cyano-4 benzyl)-2 malonate d'éthyle dans 20 $cm^3$ d'éthanol, puis 1,5 $cm^3$ d'une solution aqueuse d'acide chlorhydrique concentrée. Le mélange est hydrogéné sous pression atmosphérique, à une température voisine de 20 °C pendant 22 heures. Le mélange réactionnel est alors filtré, le catalyseur est lavé avec 2 fois 8 $cm^3$ d'éthanol. Le filtrat et les liqueurs de lavage sont réunis et concentrés à sec sous pression réduite (20 mm de mercure ; 2,7 kPa) à 30 °C. Le résidu est repris par 60 $cm^3$ d'eau distillée et la solution obtenue est agitée pendant 30 minutes. Un insoluble est séparé par filtration et le filtrat est concentré à sec sous pression réduite (1 mm de mercure ; 0,13 kPa) à 40 °C. On obtient ainsi 956 mg de chlorhydrate d'acétamido-2 (aminométhyl-4 benzyl)-2 malonate d'éthyle sous forme d'un solide blanc.

Rf : 0,28 [chromatographie sur couche mince de gel de silice ; solvant : chloroforme-méthanol-eau-acide acétique-acétate d'éthyle (respectivement 35-15-3-1,5-1 en volumes)].

Etape 3 : Préparation de l'acétamido-2 (hydroxyméthyl-4 benzyl)-2 malonate d'éthyle

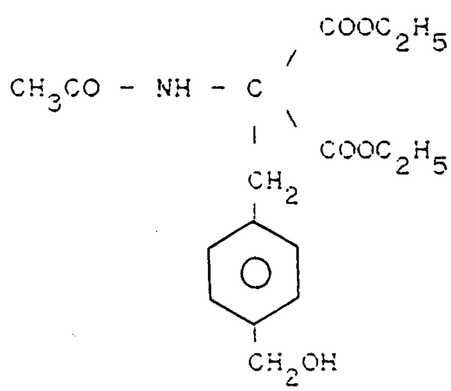

A une solution de 2,06 g de chlorhydrate d'acétamido-2 (aminométhyl-4 benzyl)-2 malonate d'éthyle dans 100 $cm^3$ d'eau distillée, on ajoute 534 mg de nitrite de sodium et on chauffe le mélange réactionnel pendant 2 heures à 110 °C. Après avoir refroidi, on extrait avec 2 fois 150 $cm^3$ d'acétate d'éthyle. Les phases organiques sont réunies et lavées successivement avec 100 $cm^3$ d'une solution aqueuse d'acide chlorhydrique 1N, puis avec 100 $cm^3$ d'une solution aqueuse de bicarbonate de sodium à 5% et, enfin, avec 100 $cm^3$ d'une solution aqueuse saturée de chlorure de sodium, puis séchées sur sulfate de sodium ; la solution est filtrée et le filtrat est évaporé à sec sous pression réduite [20 mm de mercure (2,7 kPa), puis 1 mm de mercure (0,13 kPa)] à 40 °C. On obtient ainsi 1,67 g d'acétamido-2 (hydroxyméthyl-4 benzyl)-2 malonate d'éthyle sous forme d'un solide blanc.

Rf = 0,15 [chromatographie sur couche mince de gel de silice ; solvant : hexane-acétate d'éthyle-méthanol (60-40-4 en volumes)].

Etape 4 : Préparation de l'acétamido-2 (chlorométhyl-4 benzyl)-2 malonate d'éthyle

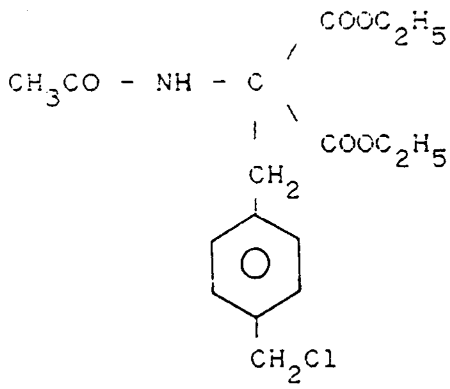

A une solution de 210 mg d'acétamido-2 (hydroxyméthyl-4 benzyl)-2 malonate d'éthyle dans 15 $cm^3$ de dichlorométhane, on ajoute 1,4 $cm^3$ de chlorure de thionyle et on chauffe le mélange réactionnel à reflux pendant 23 heures. Le dichlorométhane et l'excès de chlorure de thionyle sont éliminés par évaporation sous pression réduite [20 mm de mercure (2,7 kPa), puis 1 mm de mercure (0,13 kPa)] à 40 ° C. Le résidu est lavé avec 2 fois 3 $cm^3$ d'éther éthylique et séché sous pression réduite (20 mm de mercure ; 2,7 kPa). On obtient ainsi 161 mg d'acétamido-2 (chlorométhyl-4 benzyl)-2 malonate d'éthyle sous forme d'un solide blanc.

Rf = 0,62 [chromatographie sur couche mince de gel de silice ; solvant : dichlorométhane-méthanol (80-5 en volumes)].

Etape 5 : Préparation du produit attendu

A une solution de 3,31 g de sulfite de sodium dans 20 $cm^3$ d'eau distillée et 8 $cm^3$ d'une solution aqueuse de soude à 10%, on ajoute 1,1 g d'acétamido-2 (chlorométhyl-4 benzyl)-2 malonate d'éthyle et on chauffe le mélange réactionnel pendant 3 heures à une température voisine de 120 ° C. Après refroidissement à une température voisine de 20 ° C, on amène le mélange réactionnel à pH = 1 au moyen d'une solution aqueuse d'acide chlorhydrique 1N et on chauffe à nouveau le mélange à 120 ° C pendant 1 heure. Le mélange réactionnel est refroidi à une température voisine de 20 ° C et repris par 150 $cm^3$ d'éthanol ; les sels minéraux sont séparés par filtration et le filtrat est concentré à sec sous pression réduite (20 mm de mercure : 2,7 kPa) à 30 ° C. Le résidu obtenu est chromatographié sur une colonne de 2,5 cm de diamètre garnie de 100 g de gel de silice, en éluant avec un mélange de dichlorométhane, de méthanol, d'eau et d'acide acétique (respectivement 70-30-6-3 en volumes). On obtient ainsi 424 mg de N-acétyl sulfonatométhyl-4 phénylalanine monosodique.

Spectre de RMN du proton (270 MHz ; $D_2O$) :

1,53 ppm, s : 3H ($\underline{CH_3}$-CO-)

2,55 ppm et 2,81 ppm, dd :

2H (-C$\underline{H}_2$-CH-)

3,77 ppm, s : 2H (-C$\underline{H}_2$-$SO_3$Na)

4,11 ppm, m :

1H (-C$\underline{H}$ -COOH)

6,88 ppm, d : 2H et 6,97 ppm d : 2H (H aromatiques

Spectre de masse en ionisation FAB :

m/e = 324 ($MH^+$) (calculé : 324).

Rf = 0,16 [chromatographie sur couche mince de gel de silice ; solvant : dichlorométhane-méthanol-eau-acide acétique (70-30-6-3 en volumes)].

Dans les examples suivants, les acides aminés sont représentés par la forme abrégée conventionnelle. Les autres abréviations ont les significations suivantes :

| | |
|---|---|
| BOC = | tertiobutyloxycarbonyle |
| OBZ = | benzyloxy |
| Ac = | acétyle |
| DCC = | dicyclohexylcarbodiimide |
| HOBt = | hydroxy-1 benzotriazole |
| TFA = | acide trifluoracétique. |
| gNle = | gem diamino-1,1 pentane |
| mGly = | acide malonique |
| HONSu = | N-hydroxysuccinimide |
| Cbz = | Benzyloxycarbonyle |

Exemple 2 : Ac-L-Phe(p-$CH_2SO_3$)-NLe-Gly-Trp-NLe-Asp-PheNH$_2$
et
Exemple 3 : Ac-D-Phe(p-$CH_2SO_3Na$)-NLe-Gly-Trp-NLe-Asp-PheNH$_2$

Etape 1 : Préparation de Ac-LD-Phe(P-$CH_2SO_3Na$)-NLe-Gly-Trp-$OC_2H_5$

A une solution refroidie à 0 °C de 84,2 mg de N-acétyl sulfonatométhyl-4 LD-phénylalanine monosodique (préparée comme décrit à l'exemple 1), dans 6 cm$^3$ de diméthylformamide, on ajoute successivement 105 mg de H-NLe-Gly-Trp-$OC_2H_5$, 41,3 mg d'hydroxy-1 benzotriazole et 55,6 mg de dicyclohexylcarbodiimide. Le mélange réactionnel est agité pendant 1 heure à 0 °C et pendant une nuit à une température voisine de 20 °C. Après évaporation du diméthylformamide sous pression réduite (1 mm de mercure ; 0,13 kPa), on ajoute 10 cm$^3$ d'acétate d'éthyle. Le produit et la dicyclohexylurée précipitent. Après élimination du surnageant, on solubilise le produit dans 40 cm$^3$ d'eau, tandis que la dicyclohexylurée reste insoluble en milieu aqueux. Après filtration, la phase aqueuse est lyophilisée. On obtient ainsi 147 mg de Ac-LD-Phe(p-$CH_2SO_3Na$)-NLe-Gly-Trp-$OC_2H_5$ sous forme de produit blanc.

Rf = 0,25 [chromatographie sur couche mince de gel de silice ; éluant : dichlorométhane-méthanol-eau-acide acétique (70-30-6-3 en volumes)].

Etape 2 : Préparation de Ac-LD-Phe(p-$CH_2SO_3Na$)-NLe-Gly-Trp-OH

100 mg du produit obtenu à l'étape 1 sont dissous dans 8 cm$^3$ d'eau et 1 cm$^3$ de méthanol et la solution est refroidie à 0 °C. A cette solution, on ajoute 0,3 cm$^3$ de solution aqueuse de soude 1N. Le mélange réactionnel est agité pendant 1 heure à 0 °C et pendant 3 heures et demie à une température voisine de 20 °C. Après évaporation du méthanol, on ajoute 5 cm$^3$ d'eau et on élimine le produit non saponifié par extraction avec 6 cm$^3$ d'acétate d'éthyle. La phase aqueuse est acidifiée à 0 °C jusqu'à pH = 2 avec une solution aqueuse d'acide chlorhydrique 1N. Après lyophilisation, on obtient 90 mg de Ac-LD-Phe(p-$CH_2SO_3Na$)-Nle-Gly-Trp-OH sous forme d'un produit blanc.

Rf = 0,10 [chromatographie sur couche mince de gel de silice ; solvant : dichlorométhane-méthanol-eau-acide acétique (70-30-6-3 en volumes)].

L'absence de racémisation au niveau du résidu tryptophane, à cette étape de la synthèse, est contrôlée par RMN du $^1$H (270 MHz).

Etape 3 : Préparation de :
Ac-LD-Phe(p-$CH_2SO_3Na$)-NLe-Gly-Trp-NLe-Asp(OBZ)-PheNH$_2$

A une solution refroidie à 0 °C de 17,5 mg de H-NLe-Asp(OBZ)-Phe-NH$_2$,TFA dans 2 cm$^3$ de diméthylformamide, on ajoute successivement 5 µl de triéthylamine, 20 mg de Ac-LD-Phe(p-$CH_2SO_3Na$)-NLe-Gly-Trp-OH (préparé comme décrit à l'étape 2), 9,2 mg d'hydroxy-1 benzotriazole et 12,4 mg de dicyclohexylcarbodiimide. Le mélange réactionnel est agité pendant 1 heure à 0 °C et pendant une nuit à une température voisine de 20 °C. Après avoir évaporé le diméthylformamide sous pression réduite (1 mm de mercure ; 0,13 kPa), on ajoute 10 cm$^3$ d'acétate d'éthyle puis 25 cm$^3$ d'éther éthylique. Après agitation, décantation et prélèvement du surnageant, on lave de nouveau le produit avec 20 cm$^3$ d'éther. Après prélèvement du surnageant, le produit est séché. On obtient ainsi 26,4 mg de Ac-LD-Phe(p-$CH_2SO_3Na$)-NLe-Gly-Trp-NLe-Asp(OBZ)-PheNH$_2$ sous forme d'un produit blanc.

Rf = 0,38 pour le premier diastéréoisomère.

Rf = 0,43 pour le deuxième diastéréoisomère
[chromatographie sur couche mince de gel de silice ;
solvant : dichlorométhane-méthanol-eau-acide acétique-acétate d'éthyle (35-15-3-1,5-1 en volumes)].

Etape 4 : Préparation du mélange racémique des produits attendus

Le produit obtenu à l'étape 3 est ajouté à 14,7 mg du même produit provenant d'une autre opération et l'ensemble [soit 41,1 mg de Ac-LD-Phe(p-$CH_2SO_3Na$)-NLe-Gly-Trp-NLe-Asp(OBZ)-Phe$NH_2$ est dissous dans 8 $cm^3$ de méthanol. La solution est ajoutée à une suspension de 12 mg de catalyseur à 10% de palladium sur noir de carbone dans 2 $cm^3$ de méthanol, préalablement saturée en hydrogène pendant 1 heure et demie. L'hydrogénolyse est poursuivie pendant 2 heures à une température voisine de 20 °C, sous pression atmosphérique. Après filtration et rinçage du catalyseur (2 fois avec 6 $cm^3$ de méthanol), la solution est concentrée sous pression réduite (20 mm de mercure ; 2,7 kPa) à 30 °C. On obtient ainsi 30,7 mg de Ac-LD-Phe(p-$CH_2SO_3Na$)-NLe-Gly-Trp-NLe-Asp-Phe$NH_2$ sous forme de produit blanc.
Rf = 0,23 pour le premier diastéréoisomère
Rf = 0,20 pour le second diastéréoisomère
[chromatographie sur couche mince de gel de silice ;
solvant: dichlorométhane-méthanol-eau-acide acétique-acétate d'éthyle (35-15-3-1,5-1 en volumes)].

Etape 5 : Séparation des diastéréoisomères

La séparation des diastéréoisomères est effectuée sur 21 mg de mélange, par chromatographie sur colonne de 0,9 cm de diamètre, garnie de 15 g de silice, en éluant avec un mélange d'acétate d'éthyle, de pyridine, d'acide acétique et d'eau dans les proportions respectives suivantes : 60-20-6-11 (en volumes), en recueillant des tubes contenant 50 gouttes chacun. On obtient ainsi :

- 5,1 mg de Ac-L-Phe(p-$CH_2SO_3Na$)-NLe-Gly-Trp-NLe-Asp-Phe$NH_2$ (Exemple 2).
  Rf = 0,26 [chromatographie sur couche mince de gel de silice ; éluant : acétate d'éthyle-pyridine-acide acétique-eau (60-20-6-11 en volumes)].
  HPLC : temps de rétention = 9 minutes ; éluant : mélange de tampon phosphate de triéthylamine (0,025 M ; pH = 6,5) et d'acétonitrile (71-29 en volumes) ; débit = 1,2 $cm^3$/minute.
  Spectre de masse en ionisation FAB :
  m/e = 1054 ($MH^+$).
- 3,6 mg de Ac-D-Phe(p-$CH_2SO_3Na$)-NLe-Gly-Trp-NLe-Asp-Phe$NH_2$ (Exemple 3).
  Rf = 0,21 [chromatographie sur couche mince de gel de silice ; éluant : acétate d'éthyle-pyridine-acide acétique-eau (60-20-6-11 en volumes)].
  HPLC : temps de rétention = 7,8 minutes ; éluant : mélange de tampon phosphate de triéthylamine (0,025 M ; pH = 6,5) et d'acétonitrile (71-29 en volumes) ; débit = 1,2 $cm^3$/minute.
  Spectre de masse en ionisation FAB :
  m/e = 1054 ($MH^+$)

Exemple 4:
Boc-Tyr($SO_3Na$)-gNle-mGly-Trp-(N-Me)Nle-Asp(Na)-Phe-$NH_2$

Etape 1 : Préparation de Boc-(N-Me)Nle-OH

A une solution refroidie à 0 °C de 2,31 g de Boc-Nle-OH dans 20 ml de tétrahydrofuranne et 2,5 ml de diméthylformamide, on ajoute successivement 0,125 g d'hexaoxa-1,4,7,10,13,16 cyclooctadécane et 0,77 ml d'iodure de méthyle. Le mélange réactionnel est agité, sous azote, pendant 24 heures, à température ambiante, puis il est acidifié par de l'acide citrique 0.75M jusqu'à pH 3. La phase aqueuse est extraite par de l'éther, puis elle est rincée avec une solution aqueuse saturée de chlorure de sodium jusqu'à neutralité. La phase organique est séchée sur sulfate de sodium, filtrée et évaporée à sec sous pression réduite (20 mm de mercure ; 2,7 kPa); à une température voisine de 40 °C pour conduire à 2,32 g de Boc-(N-Me)Nle-OH, sous forme d'une huile.
Rf = 0, 54 [gel de silice ; $CHCl_3$-MeOH (85-15 en volumes)].

Etape 2 : Préparation de Boc((N-Me)Nle-Asp(OBzl)-Phe-$NH_2$

A une solution refroidie à 0 °C de 2,89 g de H-Asp(OBzl)-Phe-$NH_2$, préparé selon la méthode décrite par CHARPENTIER et al. dans "J. Med. Chem., 30, 962, 1987", dans 15 $cm^3$ de diméthylformamide et 15 $cm^3$ de dichlorométhane, on ajoute successivement 0,84 ml de triéthylamine, 1.47 g de Boc(N-Me)Nle-OH, 1,48 g de dicyclohexylcarbodiimide et 0.69 g d'hydroxysuccinimide. Le mélange réactionnel est agité pendant 1 heure à 0 °C, puis pendant une nuit à une température voisine de 20 °C. Le solide formé est éliminé par filtration, et le filtrat est concentré à sec sous pression réduite (20 mm de mercure ; 2,7 kPa), à une température voisine de 40 °C. Le résidu huileux est trituré avec de l'éther diéthylique et de l'acétate d'éthyle, puis le solide est isolé par filtration. On recueille 3,14 g de Boc-(N-Me)-Nle-Asp(OBzl)-Phe-$NH_2$, sous forme d'un solide blanc fondant à 55-57 °C.

Etape 3 : Préparation de H-(N-Me)Nle-Asp(OBzl)-Phe-$NH_2$, TFA.

On dissout 3 g de Boc-(N-Me)Nle-Asp(OBzl)-Phe-$NH_2$, dans un mélange refroidi à 0 °C de 8 $cm^3$ de chlorure de méthylène et de 8 $cm^3$ d'acide trifluoracétique, et on agite le mélange réactionnel pendant 45 minutes à 0 °C et 45 minutes à une température voisine de 20 °C. Le mélange est concentré à sec sous pression réduite (20 mm de mercure ; 2,7 kPa) à 40 °C. On obtient ainsi 2,67 g de H-(N-Me)Nle-Asp(OBzl)-Phe-$NH_2$, TFA.

Rf = 0,22 [gel de silice ; chloroforme-méthanol (9-1 en volumes)]

Etape 4 : Préparation de Boc-Trp-(N-Me)Nle-Asp(OBzl)-Phe-$NH_2$

A une solution refroidie à 0 °C de 2,5 g de H-(N-Me)Nle-Asp(OBzl)-Phe-$NH_2$, TFA dans 20 $cm^3$ de diméthylformamide contenant 0,72 ml de diisopropyléthylamine, on ajoute 2,14 g de Boc-Trp-ONp et 0,64 g de HOBt, et on agite le mélange réactionnel, sous atmosphère d'azote, pendant 30 minutes à 0 °C, puis pendant une nuit à une température voisine de 20 °C. Après évaporation du solvant sous pression réduite (1 mm de mercure ; 0,13 kPa) à 40 °C, le résidu huileux obtenu est purifié par chromatographie flash sur gel de silice, en éluant avec un mélange de chloroforme et de méthanol (98-2 en volumes). On obtient ainsi 2,54 g de Boc-Trp-(N-Me)Nle-Asp(OBzl)-Phe-$NH_2$ sous forme d'un solide blanc fondant à 98-100 °C.

Rf = 0,43 [gel de silice ; chloroforme-méthanol (9-1 en volumes)].

Etape 5 : Préparation de H-Trp-(N-Me)Nle-Asp(OBZ)-Phe-$NH_2$, TFA

On dissout 3,18 de Boc-Trp-(N-Me)Nle-Asp(OBZ)-Phe-$NH_2$ dans un mélange refroidi à 0 °C de 7 $cm^3$ de chlorure de méthylène, 7 $cm^3$ d'acide trifluoracétique et 0,5 $cm^3$ d'anisole, et on agite le mélange réactionnel sous azote pendant 45 minutes à 0 °C, et pendant 45 minutes à une température voisine de 20 °C. Les solvants sont ensuite évaporés sous pression réduite (1 mm de mercure ; 0,13 kPa) à une température voisine de 40 °C, et le résidu obtenu est précipité par de l'éther diéthylique. On obtient ainsi 2,70 g de H-Trp-(N)Me)Nle-Asp-(OBZ)-Phe-$NH_2$, TFA.

Rf = 0,20 [gel de silice ; chloroforme-méthanol (9-1 en volumes)].

Etape 6 : Préparation du N-benzyloxycarbonyl N′-malonyl-diamino-1,1 pentane (Obz-gNle-mGly-OH)

A une solution refroidie à -20 °C de 2,65 g de Cbz-Nle-OH dans 25 $cm^3$ de tétrahydrofuranne contenant 1,37 $cm^3$ de N-éthylmorpholine, on ajoute 1,03 $cm^3$ de chloroformiate d'éthyle. On agite le mélange réactionnel pendant 15 minutes à -15 °C, et on ajoute 1,3 g d'azoture de sodium en solution dans 10 $cm^3$ d'eau et on maintient sous agitation pendant 30 minutes à 0 °C. Le mélange réactionnel est extrait avec de l'acétate d'éthyle à 0 °C, et la phase organique est lavée successivement avec une solution aqueuse saturée de chlorure de sodium, une solution aqueuse saturée de bicarbonate de sodium, puis, à nouveau, par une solution aqueuse saturée de chlorure de sodium. La phase organique est séchée sur sulfate de magnésium, puis elle est filtrée et évaporée sous pression réduite (20 mm de mercure ; 2,7 kPa) à 20 °C. Le résidu huileux est repris dans 40 $cm^3$ de toluène, et il est chauffé à 80 °C pendant 10 minutes, puis il est traité par une solution de 3,12 g d'acide malonique dans 15 $cm^3$ de dioxanne. Le mélange réactionnel est alors chauffé à 80 °C pendant 30 minutes, puis il est maintenu à 0 °C pendant 1 jour. Le précipité est lavé avec du dichlorométhane, et il est purifié par chromatographie flash sur gel de silice, en éluant avec un mélange de chloroforme, de méthanol et d'acide acétique (90-5-5 en volumes), pour conduire à 1,6 g de

Boc-gNle-mGly-OH sous forme d'un solide blanc fondant à 142-145 ° C.

Rf = 0,41 [gel de silice ; chloroforme-méthanol-acide acétique (90-5-5 en volumes)].

Etape 7 : Préparation de : Cbz-gNle-mGly-Trp-(N-Me)Nle-Asp(OBZ)-Phe-$NH_2$.

A une solution refroidie à 0 ° C, de 0,46 g de H-Trp-(N-Me)Nle-Asp(OBZ)-Phe-$NH_2$ dans 3 ml de diméthylformamide, on ajoute successivement 0,066 $cm^3$ de triéthylamine, 0,18 g de Cbz-gNle-mGly-OH. 0,066 g d'hydroxysuccinimide et 0,143 g de dicyclohexylcarbodiimide. Le mélange réactionnel est agité sous azote pendant 1 heure, à 0 ° C, puis pendant une nuit à température ambiante. L'insoluble formé est éliminé par filtration, et le filtrat est condensé à sec sous pression réduite (1 mm de mercure ; 0,13 kPa). Le résidu huileux obtenu est précipité par un mélange d'éther diéthylique et d'acétate d'éthyle. On obtient ainsi, après purification par chromatographie flash sur gel de silice du précipité, en éluant avec un mélange chloroforme-méthanol (97-3 en volumes), 0,34 g de Cbz-gNle-mGly-Trp-(N-Me)Nle-Asp(OBZ)-Phe-$NH_2$. sous forme d'un solide blanc fondant à 190-193 ° C.

Rf = 0,39 [gel de silice ; chloroforme-méthanol (9-1 en volumes)].

Etape 8 : Préparation de :
H-gNle-mGly-Trp-(N-Me)Nle-Asp-Phe-$NH_2$.

A une solution de 0,32 g de Cbz-gNle-mGly-Trp-(N-Me)Nle-Asp-(OBZ)-Phe-$NH_2$. dans un mélange de 5 $cm^3$ de diméthylformamide et de 5 $cm^3$ de méthanol, on ajoute 30 mg de palladium sur charbon actif (10% en poids), et on hydrogène le mélange sous pression atmosphérique, à une température voisine de 20 ° C, pendant 4 heures. Après élimination du catalyseur par filtration, le filtrat est concentré à sec sous pression réduite (1 mm de mercure ; 0,13 kPa) à 40 ° C. On obtient ainsi 0,23 g de H-gNle-mGly-Trp-(N-Me)Nle-Asp-Phe-$NH_2$.
sous forme d'un produit solide blanc fondant à 138-140 ° C.

Rf = 0,5 [gel de silice ; acétate d'éthyle-pyridine-acide acétique-eau (40-20-6-11 en volumes)].

Etape 9 : Préparation de Boc-Tyr-gNle-mGly-Trp-(N-Me)Nle-Asp-Phe-$NH_2$

A une solution refroidie à 0 ° C de 0,2 g de H-gNle-mGly-Trp-(N-Me)Nle-Asp-Phe-$NH_2$ dans 3 $cm^3$ de diméthylformamide, on ajoute 0,12 g de Boc-Tyr-ONp et 0,05 g de HOBt et on agite le mélange réactionnel pendant 30 minutes à 0 ° C, et pendant 4 heures à une température voisine de 20 ° C. Après évaporation du solvant sous pression réduite (1 mm de mercure ; 0,13 kPa), le résidu huileux est précipité par un mélange d'acétate d'éthyle et d'éther diéthylique. On obtient ainsi 0,173 g de Boc-Try-gNle-mGly-Trp-(N-Me)Nle-Asp-Phe $NH_2$, sous forme d'un solide blanc fondant à 162-164 ° C.

Rf = 0,42 [gel de silice ; acétate d'éthyle-pyridine-acide acétique-eau (100-20-6-11 en volumes)].

Etape 10 : Préparation du produit attendu :
Boc-Tyr($SO_3$Na)-gNle-mGly-Trp-(N-Me)Nle-Asp-Phe-$NH_2$

A une solution de 0,16 g de
Boc-Tyr-gNle-mGly-Trp-(N-Me)Nle-Asp-Phe-$NH_2$ dans un mélange de 2 $cm^3$ de diméthylformamide et de 5 $cm^3$ de pyridine, on ajoute 1 g de complexe anhydride sulfuriquepyridine, et on agite le mélange réactionnel sous atmosphère d'azote pendant une nuit à une température voisine de 20 ° C. Après évaporation du solvant sous pression réduite (1 mm de mercure ; 0,13 kPa) à 40 ° C, le résidu est repris à 0 ° C par 5 $cm^3$ d'eau et la suspension est agitée pendant 3 heures à 0 ° C, en maintenant le pH à une valeur voisine de 7 au moyen d'une solution aqueuse saturée de bicarbonate de sodium. Le produit en suspension est collecté par centrifugation. Une seconde fraction de produit est obtenue par lyophilisation de la phase aqueuse et précipitation des sels minéraux par le méthanol. Ces deux fractions sont réunies et purifiées par chromatographie sur une colonne de gel de silice, en éluant avec un mélange d'acétate d'éthyle, de pyridine, d'acide acétique et d'eau (60-20-6-11 en volumes). On obtient ainsi 0,10 g de
Boc-Tyr($SO_3$Na)-gNle-mGly-Trp-(N-Me)Nle-Asp(Na)-Phe-$NH_2$ sous forme d'un solide blanc.

Rf = 0,26 [gel de silice ; acétate d'éthylepyridine-acide acétique-eau (60-20-6-11 en volumes)].

Spectre de masse en ionisation FAB :
m/e = 1150 ($MH^+$).

HPLC : temps de rétention = 19 minutes ; éluant : mélange de tampon phosphate de triéthylamine (0,025M ; pH = 6,5) et d'acétonitrile (65-35 en volumes) ; débit 1,2 $cm^3$/minute.

En opérant comme indiqué aux exemples 2 à 4, on a préparé les produits suivants selon l'une ou l'autre des voies utilisées :

Exemple 5:
Boc-Tyr($SO_3$Na)-Nle-Gly-Trp-(N-Me)Nle-Asp(Na)-Phe-$NH_2$

Rf : 0,50 [silicagel ; acétate d'éthyle-pyridine-acide acétique-eau (40-20-6-11 en volumes)]
Spectre de masse en ionisation FAB : m/e = 1150 ($MH^+$).
HPLC : temps de rétention = 28 minutes ; éluant : mélange de tampon phosphate de triéthylamine (0,025M ; pH 6,5) et d'acétonitrile (65-35 en volumes) ; débit 1,2 $cm^3$/minute.

Exemple 6 :
Boc-Tyr($SO_3$Na)-gNle-mGly-Trp-Nle-Asp(Na)-Phe-$NH_2$

Rf : 0,30 [silicagel ; acétate d'éthyle-pyridine-acide acétique-eau (80-20-6-11 en volumes)]
Spectre de masse en ionisation FAB : m/e = 1136 ($MH^+$).
HPLC : temps de rétention = 16 minutes ; éluant : mélange de tampon phosphate de triéthylamine (0,025M ; pH 6,5) et d'acétonitrile (65-35 en volumes) ; débit 1,2 ml/minute.

Exemple 7 :
Ac-L-Phe(p-$CH_2SO_3$Na)-gNle-mGly-Trp-(N-Me)Nle-Asp-Phe-$NH_2$

Rf : 0,18 [silicagel ; acétate d'éthyle-pyridine-acide acétique-eau (40-20-6-11 en volumes)]
Spectre de masse en ionisation FAB : m/e = 1068 ($MH^+$).
HPLC : temps de rétention = 7,2 minutes ; éluant : mélange de tampon phosphate de triéthylamine (0,025M ; pH 6,5) et d'acétonitrile (65-35 en volumes).

Exemple 8 : Mise en évidence de l'activité des composés des Exemples 2 à 5 et 7

1) Tests de liaison :

Les analogues de la cholécystokinine sont testés dans les expriences de compétition vis-à-vis du ligand ($^3$H)-propionyl-$CCK_8$ sur cortex et sur membranes pancréatiques de cobaye. Les protocoles expérimentaux de préparation de tissus et les conditions de liaison sont analogues à ceux décrits par PELAPRAT et al., Life Sci., 37, 2489 (1985).

Les résultats sont exprimés par les constantes d'inhibition $K_I$ dans le tableau I suivant. Les valeurs qui figurent dans ce tableau, représentent la moyenne (± e.s.m) de trois expériences indépendantes, chacune étant effectuée en triplicate. La ($^3$H)-propionyl-$CCK_8$ a été utilisée à la concentration correspondant à sa constante de dissociation c'est-à-dire, 0,2 nM pour le cerveau et 0,1 nM pour le pancréas.

TABLEAU I

| Produit testé | Test de liaison cortex de cobaye $K_I$ (en M) | Test de liaison membranes pancréatiques de cobaye $K_I$ (en M) |
|---|---|---|
| Exemple 2 | $3,2 \pm 0,59 \times 10^{-9}$ | $1,7 \pm 0,2 \times 10^{-9}$ |
| Exemple 3 | $1,6 \pm 0,21 \times 10^{-8}$ | $1,9 \pm 0,4 \times 10^{-8}$ |
| Exemple 4 | $0,11 \pm 0,02 \times 10^{-9}$ | $1,06 \pm 0,07 \times 10^{-9}$ |
| Exemple 5 | $0,15 \pm 0,02 \times 10^{-9}$ | $78 \pm 6 \times 10^{-9}$ |
| Exemple 7 | $1,3 \pm 0,53 \times 10^{-9}$ | $93 \pm 4 \times 10^{-9}$ |

2) Test de libération d'amylase pancréatique :

La sécrétion d'amylase est mesurée après incubation pendant 30 minutes à 37 °C d'acini pancréatiques en présence des produits à étudier, selon le protocole expérimental décrit par PEIKIN et al., Am. J. Physiol., 235 (6), E 743-E 749, (1978). L'activité amylasique est déterminée à l'aide du réactif Phadebas (Pharmacia), selon la méthode décrite par CESKA et al., Experientia, 25, 555 (1969). Les résultats observés

figurent dans le tableau II suivant. Les valeurs indiquées dans ce tableau représentent la moyenne de trois expériences indépendantes, chacune effectuées en triplicate.

TABLEAU II

| Produit testé | Sécrétion d'amylase d'acini pancréatiques de cobaye Activité agoniste $EC_{50}$ (M) |
|---|---|
| Exemple 2 | $3,3 \pm 0,9 \times 10^{-10}$ |
| Exemple 3 | $1,6 \pm 0,4 \times 10^{-9}$ |
| Exemple 4 | $0,35 \pm 0,07 \times 10^{-9}$ |
| Exemple 5 | $377 \pm 118 \times 10^{-9}$ |
| Exemple 7 | $642 \pm 91 \times 10^{-9}$ |

3) Test d'activité contracturante sur l'iléon de cobaye

Ce test est réalisé selon la méthode décrite par HUTCHINSON et al., Eur. J. Pharmacol., 69, 87 (1981). Des bandelettes d'iléon de cobaye de la partie terminale sont rapidement prélevées et fixées dans la cuve d'un capteur isométrique contenant 25 $cm^3$ d'une solution de Tyrode. La solution est maintenue à 37 °C tout en faisant barboter bulle à bulle un gaz composé de 95% d'oxygène et de 5% de $CO_2$. Les composés à étudier sont testés dans les conditions décrites par RUIZ-GAYO et al., Peptides, 6, 415, (1985).

Les résultats observés sont reportés dans le tableau III. Les valeurs indiquées dans ce tableau représentent la moyenne (± e.s.m.) de trois expériences indépendantes.

TABLEAU III

| Produit testé | Activité contracturante sur iléon de cobaye Activité agoniste $EC_{50}$ (M) |
|---|---|
| Exemple 2 | $3,8 \pm 0,3 \times 10^{-9}$ |
| Exemple 3 | $1,8 \pm 0,4 \times 10^{-8}$ |
| Exemple 4 | $1,0 \pm 0,1 \times 10^{-9}$ |
| Exemple 5 | >1000 |
| Exemple 7 | >1000 |

Exemple 9 : Test de stabilité enzymatique sur les composés des exemples 4, 5 et 7

La résistance à la dégradation enzymatique de ces analogues a été testée dans des membranes brutes de cerveau de rat qui contiennent à la fois l'activité aminopeptidasique, thiolprotéasique et enképhalinasique [MATSAS et al. FEBS. Lett. 175, 124 (1984) ; Mc DERMOTT et al. Neurochem. Int. 5, 641 (1983)]. Ces composés ont été testés suivant le protocole décrit précédemment par DURIEUX et al. Neuropeptides 7, 1, (1986). Les valeurs obtenues (temps de 1/2 vie) figurent dans le tableau IV.

TABLEAU IV

| Produit testé | Temps de 1/2 vie |
|---|---|
| CCK8 | 45 minutes |
| Exemple 4 | >180 minutes |
| Exemple 5 | >250 minutes |
| Exemple 7 | >250 minutes |

Exemple 10

On prépare une solution injectable contenant 2 mg de produit actif ayant la composition suivante :

| | |
|---|---|
| - N-acétyl sulfonatométhyl-4 phénylalaninate monosodique<br>- Eau distillée | 2 mg<br>Q.S.P. 2 $cm^3$ |

Exemple 11

On prépare une solution injectable contenant 1 mg de produit actif ayant la composition suivante :

| | |
|---|---|
| - Ac-L-Phe(p-$CH_2SO_3Na$)-NLe-Gly-Trp-NLe-Asp-Phe-$NH_2$<br>- Eau distillée | 1,03 mg<br>Q.S.P. 2 $cm^3$ |

Exemple 12 :

On prépare une solution injectable contenant 1 mg de produit actif ayant la composition suivante :

| | |
|---|---|
| - Boc-Tyr($SO_3Na$)-gNle-mGly-Trp-(N-Me)Nle-Asp(Na)-Phe-$NH_2$<br>- Eau distillée | 1 mg<br>Q.S.P. 2 $cm^3$ |

Exemple 13 :

On prépare une solution injectable contenant 1 mg de produit actif ayant la composition suivante :

| | |
|---|---|
| - Al-L-Phe(p-$CH_2SO_3Na$)-gNle-mGly-Trp-(N-Me)Nle-Asp-Phe-$NH_2$<br>- Eau distillée | 1 mg<br>Q.S.P. 2 $cm^3$ |

**Revendications**

**Revendications pour les Etats contractants suivants : AT, BE, CH, DE, FR, GB, IT, LI, LU, NL, SE**

**1.** Composés chimiques représentés par la formule (I):

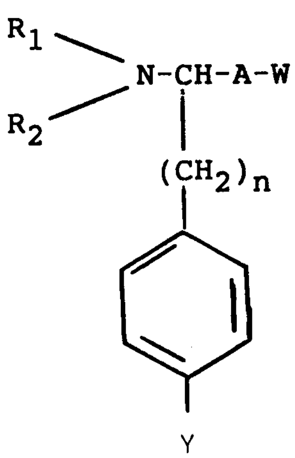

(I)

dans laquelle :

- $R_1$ représente un atome d'hydrogène
- $R_2$ représente un radical protecteur de fonction amine du type acyle ou du type uréthanne ,
- A représente - CO
- Y représente un reste choisi parmi ceux de formule :

-OSO2OR3 et
$-(CH_2)_m-SO_2OR_3$

où
* $R_3$ représente un atome d'hydrogène ou un métal alcalin;
* m est I ,
n est O ou I,
W représente :

B - D - Trp - E- Asp - PHeNHQ,

où
* B et E, représentent un reste norleucine, ou de ses dérivés N-méthylés correspondants ,
* D représente un reste glycine ;
* Q représente un hydrogène

lesdits composés se présentant sous les formes D, L ou DL, ainsi que leurs mélanges et leurs sels pharmaceutiquement acceptables.

**2.** Composé chimique selon la revendication 1, caractérisé par le fait que le radical protecteur de fonction amine du type acyle entrant dans la définition de $R_2$ est un radical formyle, acétyle ,chloroacétyle, trifluoracétyle, propionyle, butyryle, isobutyryle, $\gamma$-chlorobutyryle, oxalyle, succinyle, glutamyle. pyroglutamyle, phtalyle, p-toluènesulfonyle.

**3.** Composé chimique selon la revendication 1, caractérisé par le fait que le radical protecteur de fonction amine du type uréthanne entrant dans la définition de $R_2$ est un radical tert-butyloxycarbonyle, isopropyloxycarbonyle, éthoxycarbonyle, allyloxycarbonyle, benzyloxycarbonyle, mono- ou polyhalobenzyloxycarbonyle, nitrobenzyloxycarbonyle.

**4.** Procédé de préparation du composé tel que défini à la revendication 1, caractérisé par le fait que :
- on réalise le couplage d'un peptide de formule générale (XIII):

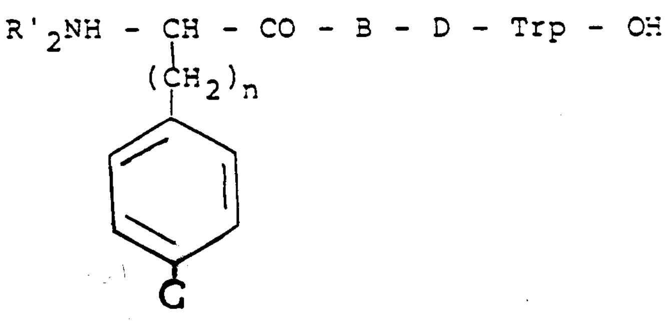

(XIII)

ou un dérivé activé de ce peptide
où:
- $R'_2$ représente un radical protecteur d'amine tel que défini dans la revendication 1 pour $R_2$; et
- G représente OH, ou un radical de formule $-(CH_2)_mSO_2OR_3$, telle que définie en relation avec la formule (I) et
- les autres symboles sont tels que définis dans la revendication 1 en relation avec la formule (I);

avec le tripeptide de formule (XIV):

$H\text{-}E\text{-}Asp\text{-}PheNH_2$ (XIV)

où E est tel que défini dans la revendication 1, ce tripeptide étant éventuellement protégé,

- puis on élimine le (ou les) groupement(s) protecteur (s) , et on isole le produit obtenu à l'état libre ou à l'état de sel, et on le transforme éventuellement en un autre produit tel que défini ci-dessus, par toute méthode connue en soi, et on transforme, si on le désire, le produit final en sel pharmaceutiquement acceptable ou en produit à l'état libre selon le cas.

**5.** Procédé selon la revendication 4, caractérisé par le fait que le produit de formule générale (XIII) est obtenu par couplage du peptide de formule (XV):

H-B-D-Trp-OH (XV)

où B et D sont tels que définis dans la revendication 1, sur un amino-acide de formule générale (XVI):

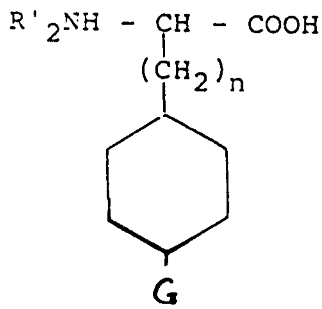

(XVI)

dans laquelle :
- G représente OH, ou un radical de formule $-(CH_2)_m-SO_2OR_3$, telle que définie en relation avec la formule (I) ;
- les sygles $R'_2$ et n sont tels que définis précédemment , en relation avec la formule (I).

**6.** Procédé de préparation des composés de formule (I) conformes à la revendication 1, caractérisé par le fait que ces composés sont préparés par couplage d'un peptide de formule générale :

H-B-D-Trp-E-Asp($OR_5$)-Phe-NH-Q (XVII)

où:
- B, D, E et Q sont tels que définis dans la revendication 1 et
- $R_5$ représente un radical protecteur d'acide carboxylique , et, plus particulièrement, un groupe tert.butyle ou benzyle.

sur un composé de la formule (XVI) , telle que définie ci-dessus.

**7.** Composition pharmaceutique, caractérisée par le fait qu'elle contient au moins un composé tel que défini à l'une des revendications 1 à 3, en association avec un ou plusieurs diluants ou adjuvants compatibles ou pharmaceutiquement acceptables.

**8.** Composition selon la revendication 7, caractérisée par le fait qu'elle est destinée au traitement des maladies nerveuses d'origine centrale, à pallier le vieillissement des cellules nerveuses, au traitement de la douleur, et qu'elle est appliquée comme anorexigène, et comme agent stimulant la motilité intestinale.

**Revendications pour l'Etat contractant suivant : GR**

1. Composés chimiques représentés par la formule (I):

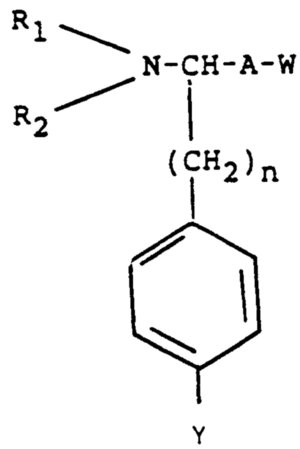

(I)

dans laquelle :
- $R_1$ représente un atome d'hydrogène
- $R_2$ représente un radical protecteur de fonction amine du type acyle ou du type uréthanne ,
- A représente - CO
- Y représente un reste choisi parmi ceux de formule :

  -OSO2OR3 et
  $-(CH_2)_m-SO_2OR_3$

  où
  * $R_3$ représente un atome d'hydrogène ou un métal alcalin;
  * m est I ,
    n est O ou I,
    W représente :

    B - D - Trp - E- Asp - PHeNHQ,

    où
    * B et E, représentent un reste norleucine, ou de ses dérivés N-méthylés correspondants ,
    * D représente un reste glycine ;
    * Q représente un hydrogène

lesdits composés se présentant sous les formes D, L ou DL, ainsi que leurs mélanges et leurs sels pharmaceutiquement acceptables .

2. Composé chimique selon la revendication 1, caractérisé par le fait que le radical protecteur de fonction amine du type acyle entrant dans la définition de $R_2$ est un radical formyle, acétyle ,chloroacétyle, trifluoracétyle, propionyle, butyryle, isobutyryle, $\gamma$-chlorobutyryle, oxalyle, succinyle, glutamyle, pyroglutamyle, phtalyle, p-toluènesulfonyle.

3. Composé chimique selon la revendication 1, caractérisé par le fait que le radical protecteur de fonction amine du type uréthanne entrant dans la définition de $R_2$ est un radical tert-butyloxycarbonyle, isopropyloxycarbonyle, éthoxycarbonyle, allyloxycarbonyle, benzyloxycarbonyle, mono- ou polyhalobenzyloxycarbonyle, nitrobenzyloxycarbonyle.

4. Procédé de préparation du composé tel que défini à la revendication 1, caractérisé par le fait que :

- on réalise le couplage d'un peptide de formule générale (XIII):

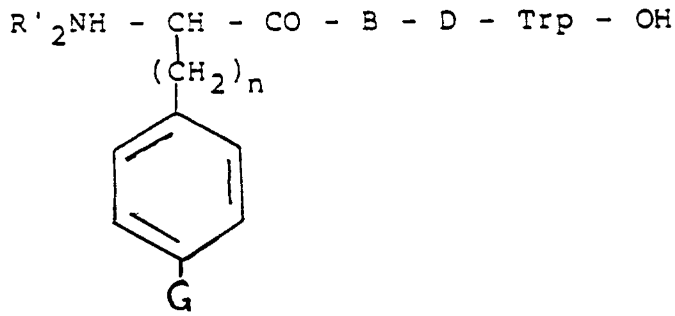

(XIII)

ou un dérivé activé de ce peptide

où:

- R'$_2$ représente un radical protecteur d'amine tel que défini dans la revendication 1 pour R$_2$; et
- G représente OH, ou un radical de formule $-(CH_2)_mSO_2OR_3$, telle que définie en relation avec la formule (I) et
- les autres symboles sont tels que définis de la rev° 1 en relation avec la formule (I):

avec le tripeptide de formule (XIV):

H-E-Asp-PheNH$_2$ (XIV)

où E est tel que défini de la rev° 1, ce tripeptide étant éventuellement protégé,

- puis on élimine le (ou les) groupement(s) protecteur (s) , et on isole le produit obtenu à l'état libre ou à l'état de sel, et on le transforme éventuellement en un autre produit tel que défini ci-dessus, par toute méthode connue en soi, et on transforme, si on le désire, le produit final en sel pharmaceutiquement acceptable ou en produit à l'état libre selon le cas.

**5.** Procédé selon la revendication 4, caractérisé par le fait que le produit de formule générale (XIII) est obtenu par couplage du peptide de formule (XV):

H-B-D-Trp-OH (XV)

où B et D sont tels que définis de la rev° 1, sur un amino-acide de formule générale (XVI):

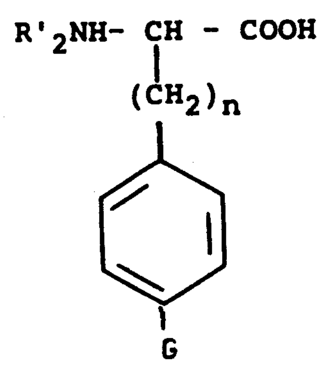

(XVI)

dans laquelle :

- G représente OH, ou un radical de formule $-(CH_2)_m-SO_2OR_3$, telle que définie en relation avec la formule (I) ;
- les sigles R'$_2$ et n sont tels que définis précédemment , en relation avec la formule (I).

**6.** Procédé de préparation des composés de formule (I) conformes à la revendication 1, caractérisé par le fait que ces composés sont préparés par couplage d'un peptide de formule générale :

H-B-D-Trp-E-Asp($OR_5$)-Phe-NH-Q (XVII)

où
- B, D, E et Q sont tels que définis dans la revendication 1; et
- $R_5$ représente un radical protecteur d'acide carboxylique , et , plus particulièrement , un groupe tert. butyle ou benzyle

sur un composé de la formule (XVI) , telle que définie ci-dessus .

**7.** Procédé de préparation d'une composition pharmaceutique , caractérisée par le fait que l'on associe au moins un composé tel que défini à l'une des revendications 1 à 3, avec un ou plusieurs diluants ou adjuvants compatibles ou pharmaceutiquement acceptable .

**8.** Procédé selon la revendication 7, caractérisé par le fait que la composition est destinée au traitement des maladies nerveuses d'origine centrale, à pallier le vieillissement des cellules nerveuses, au traitement de la douleur, et qu'elle est appliquée comme anorexigène, et comme agent stimulant la motilité intestinale.

**Revendications pour l'Etat contractant suivant : ES**

**1.** Procédé de préparation des composés chimiques représentés par la formule (I) :

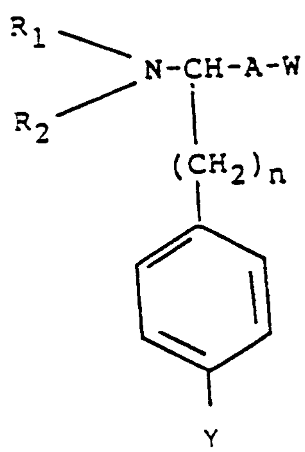

(I)

dans laquelle :
- $R_1$ représente un atome d'hydrogène
- $R_2$ représente un radical protecteur de fonction amine du type acyle ou du type uréthanne ,
- A représente - CO
- Y représente un reste choisi parmi ceux de formule :

$-OSO_2OR_3$ et
$-(CH_2)_m-SO_2OR_3$

où
* $R_3$ représente un atome d'hydrogène ou un métal alcalin ;
* m est 1 ,

n est O ou 1 ,

W représente :

B - D - Trp - E - Asp - PHeNHQ,

où
* B et E, représentent un reste norleucine, ou de ses dérivés N-méthylés correspondants ,
* D représente un reste glycine ;

* Q représente un hydrogène

lesdits composés se présentant sous les formes D , L ou DL, ainsi que de leurs mélanges et leurs sels pharmaceutiquement acceptables ,

caractérisé par le fait que :

- on réalise le couplage d'un peptide de formule générale (XIII):

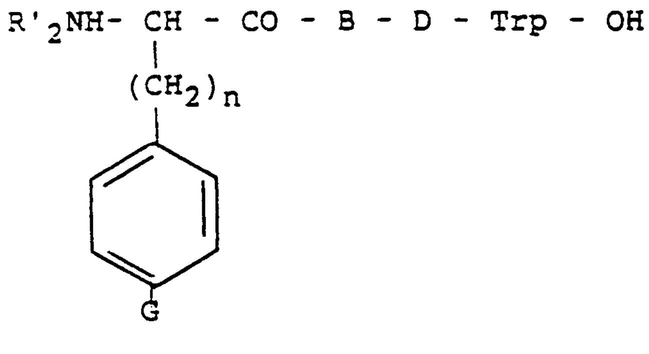

(XIII)

ou un dérivé activé de ce peptide , où :

- R'$_2$ représente un radical protecteur d'amine tel que défini ci-dessus pour $R_2$ ; et
- G représente OH, ou un radical de formule $(CH_2)_m$ $SO_2OR_3$ telle que définie en relation avec la formule ( I ) et
- les autres symboles sont tels que définis ci-dessus en relation avec la formule ( I ) ; avec le tripeptide de formule (XIV) :

H-E-Asp-PheNH$_2$ (XIV)

où E est tel que défini ci-dessus , ce tripeptide étant éventuellement protégé ,

- puis on élimine le ( ou les ) groupement (s) protecteur (s) , et on isole le produit obtenu à l'état libre ou à l'état de sel , et on le transforme éventuellement en un autre produit tel que défini ci-dessus , par toute méthode connue en soi , et on transforme , si on le désire, le produit final en sel pharmaceutiquement acceptable ou en produit à l'état libre selon le cas .

**2.** Procédé selon la revendication 1 , caractérisé par le fait que le produit de formule générale (XIII) est obtenu par couplage du peptide de formule (XV) :

H-B-D-Trp-OH

où B et D sont tels que définis dans la revendication 1 , sur un aminoacide de formule générale (XVI) :

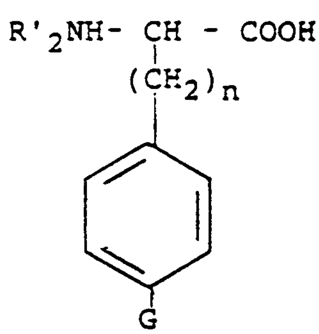

(XVI)

dans laquelle :

- G représente OH, ou un radical de formule $(CH2)_m$-$SO_2OR_3$ , telle que définie en relation avec la formule (I) ;
- les sigles R'$_2$ et n sont tels que définis précédemment, en relation avec la formule (I).

**3.** Procédé de préparation des composés de formule (I) tel que définie dans la revendication 1 , caractérisé par le fait qu'on réalise le couplage d'un peptide de formule générale :

H-B-D-Trp-E-Asp($OR_5$)-Phe-NH-Q (XVII)

où
- B, D, E et Q sont tels que définis dans la revendication 1 ; et
- $R_5$ représente un radical protecteur d'acide carboxylique, et , plus particulièrement , un groupe tert.butyle ou benzyle ,

sur un composé de la formule (XVI) , telle que définie à la revendication 2.

**4.** Procédé selon l'une des revendications 1 et 3 , caractérisé par le fait que le radical protecteur de fonction amine du type acyle entrant dans la définition de $R_2$ est un radical formyle , acétyle, chloroacétyle, trifluoracétyle , propionyle, butyryle, isobutyryle , $\gamma$-chlorobutyryle, oxalyle, succinyle, glutamyle , pyroglutamyle , phtalyle, p-toluènesulfonyle .

**5.** Procédé selon l'une des revendications 1 et 3 , caractérisé par le fait que le radical protecteur de fonction amine du type uréthanne entrant dans la définition de $R_2$ est un radical tert-butyloxycarbonyle, isopropyloxycarbonyle ,éthoxycarbonyle, allyloxycarbonyle , benzyloxycarbonyle , mono- ou polyhalobenzyloxycarbonyle , nitrobenzyloxycarbonyle .

**6.** Procédé de préparation d'une composition pharmaceutique selon lequel on associe un composé tel que préparé par le procédé selon l'une quelconque des revendications 1 à 3 avec un ou plusieurs diluants ou adjuvants compatibles ou pharmaceutiquement acceptables .

**7.** Procédé selon la revendication 6 , caractérisé en ce que la composition est destinée au traitement des maladies nerveuses d'origine centrale, à pallier le vieillissement des cellules nerveuses , au traitement de la douleur , et qu'elle est appliquée comme anorexigène, et comme agent stimulant la motilité intestinale .

**Claims**
**Claims for the following Contracting States : AT, BE, CH, DE, FR, GB, IT, LI, LU, NL, SE**

**1.** Chemical compounds represented by the formula:

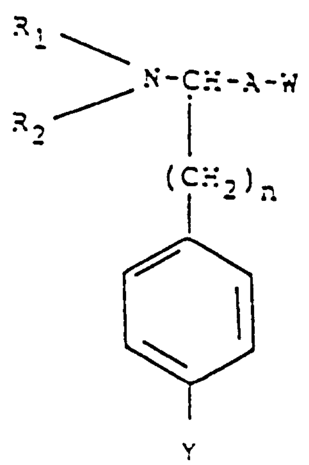

(I)

in which :
- $R_1$ represents a hydrogen atom,
- $R_2$ represents a radical which protects the amine function , of the acyl type or the urethane type,
- A represents -CO
- Y represents a residue chosen from those of formula :

- $OSO_2OR_3$ and

- $(CH_2)m-SO_2OR_3$

where
* $R_3$ represents a hydrogen atom or an alcalin metal, and
* m is 1 ,
* n is 0 or 1
- W represents :

-B-D-Trp-E-Asp-PheNHQ,

where:
* B and E represent a norleucine residue, or one of its corresponding N-methylated derivatives,
* D represents a glycine residue;
* Q represents hydrogen

the said compounds being in the D, L or DL forms, as well as their mixtures and their pharmaceutically acceptable salts.

**2.** Chemical compound as claimed in claim 1, characterized in that the radical of the acyl type which protects the amine function, and falls within the definition or $R_2$, is a formyl, acetyl, chloroacetyl, trifluoroacetyl, propionyl, butyryl, isobutyryl, gamma-chlorobutyryl, oxalyl, succinyl, glutamyl, pyroglutamyl, phthalyl or p-toluene-sulfonyl radical.

**3.** Chemical compound as claimed in claim 1, characterized in that the radical of the urethane type which protects the amine function, and falls within the definition of $R_2$ is a tert-butyloxycarbonyl, isopropyloxycarbonyl, ethoxycarbonyl, allyloxycarbonyl, benzyloxycarbonyl, mono- or polyhalobenzyloxycarbonyl or nitrobenzyloxycarbonyl radical.

**4.** Process for the preparation of the compound as defined in claim 1, characterized in that :
- a peptide of general formula (XIII):

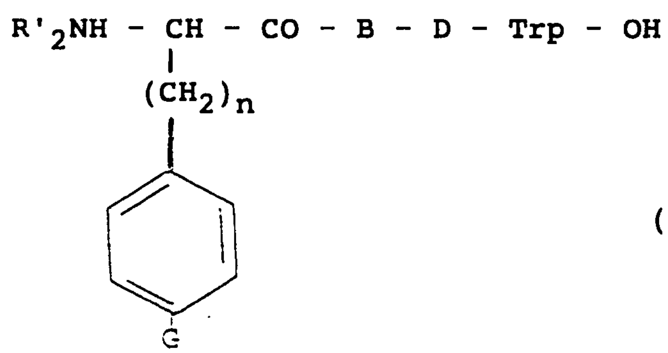

(XIII)

or an activated derivative of that peptide where :
- $R'_2$ represents an amine-protecting radical such as defined for $R_2$ in claim 1; and
- G represents OH, or a radical of formula $-(CH_2)_m SO_2OR_3$, as defined with respect to formula (I); and
- the other symbols are as defined in claim 1 with respect to formula (I);

is coupled with the tripeptide of formula (XIV)

$H-E-Asp-PheNH_2$ (XIV)

where E is as defined in claim 1, this tripeptide optionally being protected,
- then the protective group (s) are removed, and the product obtained is isolated in the free state of in the state of a salt, and is optionally converted to another product as defined above, by any method known per se, and the final product is converted, if desired , to a pharmaceutically acceptable salt or a product in the free state, depending on the circumstances.

**5.** Process as claimed in claim 4, characterized in that the product of general formula (XIII) is obtained by coupling the peptide of formula (XV):

H-B-D-Trp-OH (XV)

where B and D are as defined in claim 1 with an amino acid of general formula (XVI):

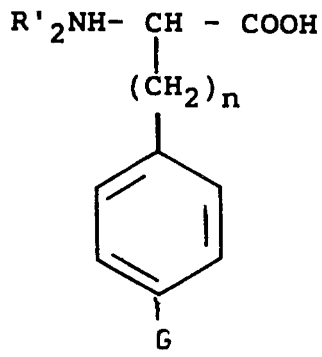

(XVI)

in which :
- G represents OH, or a radical of formula :

  $-(CH_2)_m-SO_2OR_3$

  as defined with respect to formula (I);
- the symbols $R'_2$ and n are as defined above, with respect to formula (I).

**6.** Process for the preparation of compounds of formula (I) corresponding to claim 1, characterized in that these compounds are prepared by coupling a peptide of general formula:

H-B-D-Trp-E-Asp($OR_5$)-Phe-NH-Q (XVII)

where :
- B, D, E and Q are as defined in claim 1; and
- $R_5$ represents a carboxylic acid protecting radical, and, more particularly, a tert-butyl or benzyl group

with a compound of formula (XVI), as defined hereabove.

**7.** Pharmaceutical composition characterized in that it contains at least one compound as defined in one of claims 1 to 3, in association with one or more diluents or adjuvants which are compatible or pharmaceutically acceptable.

**8.** Composition as claimed in claim 7, characterized in that it is intended for the treatment of nervous diseases of central origin, for palliation of the ageing of nervous cells, for the treatment of pain, and is applied as an anorexia- producing agent and as an agent for the stimulation of intestinal motility.

**Claims for the following Contracting State : GR**

1. Chemical compounds represented by the formula:

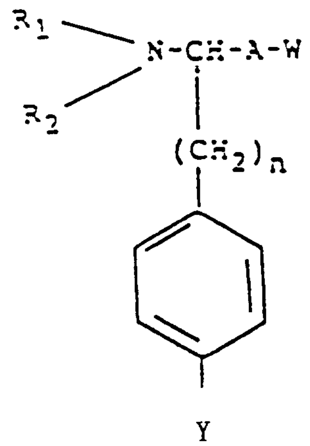

(I)

in which :
- $R_1$ represents a hydrogen atom,
- $R_2$ represents a radical which protects the amine function , of the acyl type or the urethane type,
- A represents -CO
- Y represents a residue chosen from those of formula :

  - $OSO_2OR_3$ and
  - $(CH_2)m-SO_2OR_3$

  where
  * $R_3$ represents a hydrogen atom or an alcalin metal, and
  * m is 1 ,
  * n is 0 or 1
  - W represents :

    -B-D-Trp-E-Asp-PheNHQ,

    where:
    * B and E represent a norleucine residue, or one of its corresponding N-methylated derivatives,
    * D represents a glycine residue;
    * Q represents hydrogen

the said compounds being in the D, L or DL forms, as well as their mixtures and their pharmaceutically acceptable salts.

2. Chemical compound as claimed in claim 1, characterized in that the radical of the acyl type which protects the amine function, and falls within the definition or $R_2$, is a formyl, acetyl, chloroacetyl, trifluoroacetyl, propionyl, butyryl, isobutyryl, gamma-chlorobutyryl, oxalyl, succinyl, glutamyl, pyroglutamyl, phthalyl or p-toluene-sulfonyl radical.

3. Chemical compound as claimed in claim 1, characterized in that the radical of the urethane type which protects the amine function, and falls within the definition of $R_2$ is a tert-butyloxycarbonyl, isopropyloxycarbonyl, ethoxycarbonyl, allyloxycarbonyl, benzyloxycarbonyl, mono- or polyhalobenzyloxycarbonyl or nitrobenzyloxycarbonyl radical.

4. Process for the preparation of the compound as defined in claim 1, characterized in that :

- a peptide of general formula (XIII):

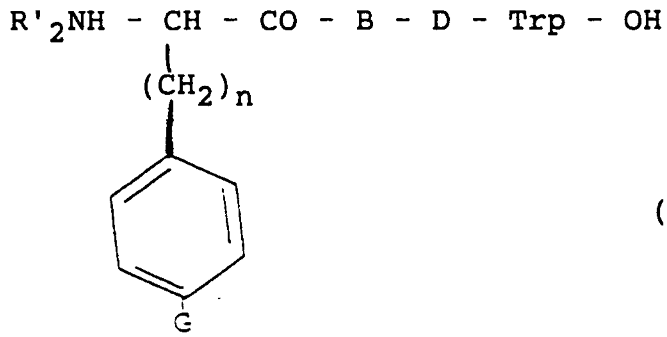

(XIII)

or an activated derivative of that peptide where :
- $R'_2$ represents an amine-protecting radical such as defined for $R_2$ in claim 1; and
- G represents OH, or a radical of formula $-(CH_2)_m\ SO_2OR_3$, as defined with respect to formula (I); and
- the other symbols are as defined in claim 1 with respect to formula (I);

is coupled with the tripeptide of formula (XIV)

H-E-Asp-Phe$NH_2$ (XIV)

where E is as defined in claim 1, this tripeptide optionally being protected,
- then the protective group (s) are removed, and the product obtained is isolated in the free state of in the state of a salt, and is optionally converted to another product as defined above, by any method known per se, and the final product is converted, if desired , to a pharmaceutically acceptable salt or a product in the free state, depending on the circumstances.

**5.** Process as claimed in claim 4, characterized in that the product of general formula (XIII) is obtained by coupling the peptide of formula (XV):

H-B-D-Trp-OH (XV)

where B and D are as defined in claim 1 with an amino acid of general formula (XVI):

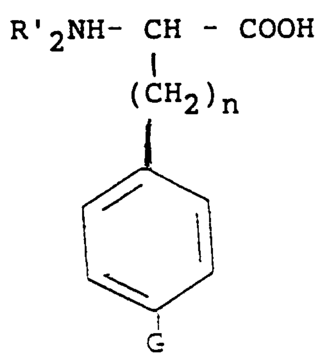

(XVI)

in which :
- G represents OH, or a radical of formula :

$-(CH_2)_m-\ SO_2OR_3$

as defined with respect to formula (I);
- the symbols $R'_2$ and n are as defined above, with respect to formula (I).

**6.** Process for the preparation of compounds of formula (I) corresponding to claim 1, characterized in that these compounds are prepared by coupling a peptide of general formula:

H-B-D-Trp-E-Asp($OR_5$)-Phe-NH-Q (XVII)

where :
- B, D, E and Q are as defined in claim 1; and
- $R_5$ represents a carboxylic acid protecting radical, and, more particularly, a tert-butyl or benzyl group

with a compound of formula (XVI), as defined hereabove.

**7.** Process for the preparation of a pharmaceutical composition characterized in that at least one compound as defined in one of claims 1 to 3, is associated with one or more diluents or adjuvants which are compatible or pharmaceutically acceptable.

**8.** Process as as claimed in claim 7, characterized in that the composition is intended for the treatment of nervous diseases of central origin, for palliation of the ageing of nervous cells, for the treatment of pain, and is applied as an anorexia-producing agent and as an agent for the stimulation of intestinal motility.

**Claims for the following Contracting State : ES**

**1.** Process for the preparation of chemical compounds represented by the formula:

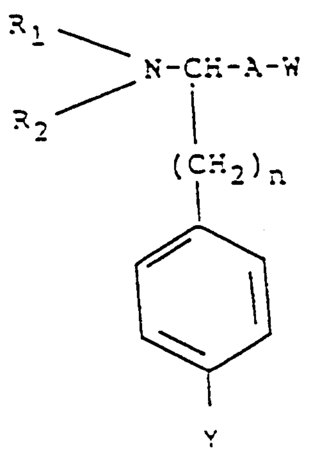

(I)

in which :
- $R_1$ represents a hydrogen atom,
- $R_2$ represents a radical which protects the amine function , of the acyl type or the urethane type,
- A represents -CO
- Y represents a residue chosen from those of formula :

  - $OSO_2OR_3$ and
  - $(CH_2)$m-$SO_2OR_3$

  where
  * $R_3$ represents a hydrogen atom or an alcalin metal, and
  * m is 1 ,
  * n is 0 or 1
  - W represents :

  -B-D-Trp-E-Asp-PheNHQ,

  where:
  * B and E represent a norleucine residue, or one of its corresponding N-methylated derivatives,

* D represents a glycine residue;
* Q represents hydrogen

the said compounds being in the D, L or DL forms, as well as their mixtures and their pharmaceutically acceptable salts, characterized in that :

- a peptide of general formula (XIII):

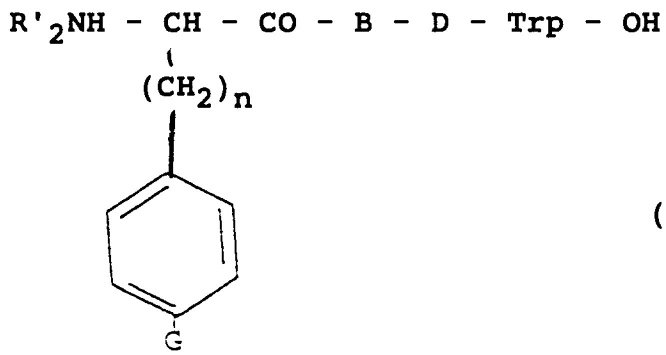

(XIII)

or an activated derivative of that peptide where :

- $R'_2$ represents an amine-protecting radical such as defined for $R_2$ in claim 1; and
- G represents OH, or a radical of formula $-(CH_2)_m\ SO_2OR_3$, as defined with respect to formula (I); and
- the other symbols are as defined in claim 1 with respect to formula (I);

is coupled with the tripeptide of formula (XIV)

$H\text{-}E\text{-}Asp\text{-}PheNH_2$ (XIV)

where E is as defined in claim 1, this tripeptide optionally being protected,

- then the protective group (s) are removed, and the product obtained is isolated in the free state of in the state of a salt, and is optionally converted to another product as defined above, by any method known per se, and the final product is converted, if desired , to a pharmaceutically acceptable salt or a product in the free state, depending on the circumstances.

**2.** Process as claimed in claim 1, characterized in that the product of general formula (XIII) is obtained by coupling the peptide of formula (XV):

H-B-D-Trp-OH (XV)

where B and D are as defined in claim 1 with an amino acid of general formula (XVI):

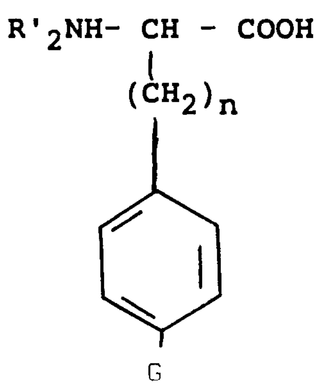

(XVI)

in which :

- G represents OH, or a radical of formula :

$-(CH_2)_m- SO_2OR_3$

as defined with respect to formula (I);
- the symbols $R'_2$ and n are as defined above, with respect to formula (I).

**3.** Process for the preparation of compounds of formula (I) as defined in claim 1, characterized in that these compounds are prepared by coupling a peptide of general formula:

H-B-D-Trp-E-Asp($OR_5$)-Phe-NH-Q (XVII)

where :
- B, D, E and Q are as defined in claim 1; and
- $R_5$ represents a carboxylic acid protecting radical, and, more particularly, a tert-butyl or benzyl group with a compound of formula (XVI), as defined in claim 2.

**4.** Process as claimed in one of claims 1 to 3 characterized in that the radical of the acyl type which protects the amine function, and falls within the definition or $R_2$, is a formyl, acetyl, chloroacetyl, trifluoroacetyl, propionyl, butyryl, isobutyryl, gamma-chlorobutyryl, oxalyl, succinyl, glutamyl, pyroglutamyl, phthalyl or p-toluene-sulfonyl radical.

**5.** Process as claimed in one of claims 1 to 3, characterized in that the radical of the urethane type which protects the amine function, and falls within the definition of $R_2$ is a tert-butyloxycarbonyl, isopropyloxycarbonyl, ethoxycarbonyl, allyloxycarbonyl, benzyloxycarbonyl, mono- or polyhalobenzyloxycarbonyl or nitrobenzyloxycarbonyl radical.

**6.** Process for the preparation of a pharmaceutical composition characterized in that at least one compound as prepared by the process according to one of claims 1 to 3, is associated with one or more diluents or adjuvants which are compatible or pharmaceutically acceptable.

**7.** Process as claimed in claim 6, characterized in that the composition is intended for the treatment of nervous diseases of central origin, for palliation of the ageing of nervous cells, for the treatment of pain, and is applied as an anorexia- producing agent and as an agent for the stimulation of intestinal motility.

**Patentansprüche**

**Patentansprüche für folgende Vertragsstaaten : AT, BE, CH, DE, FR, GB, IT, LI, LU, NL, SE**

**1.** Chemische Verbindungen, dargestellt durch die Formel (I):

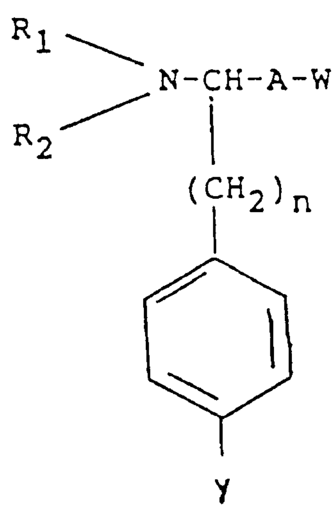

(I)

in der:
- $R_1$ ein Wasserstoffatom darstellt

- $R_2$ eine Schutzgruppe der Aminfunktion vom Typ Acyl oder vom Typ Urethan darstellt
- A -CO darstellt
- Y ein Rest ist, der ausgewählt ist aus solchen der Formel:
- $OSO_2OR_3$ und
- $(CH_2)_m$-$SO_2OR_3$ , worin
  - * $R_3$ ein Wasserstoffatom oder ein Alkalimetall darstellt;
  - * m 1 ist

  n 0 oder 1 ist,

  W : B-D-Trp-E-Asp-PheNHQ darstellt, worin
  - * B und E einen Norleucin-Rest oder einen seiner entsprechenden N-methylierten Derivate darstellen,
  - * D ein Glycinrest ist,
  - * Q Wasserstoff darstellt,

wobei die genannten Verbindungen in den Formen D,L oder DL, vorliegen, ebenso wie deren Mischungen und ihren pharmazeutisch akzeptierbaren Salzen.

**2.** Chemische Verbindung gemäß Anspruch 1, dadurch gekennzeichnet, daß die durch $R_2$ definierte Schutzgruppe der Aminfunktion vom Typ Acyl, ein Formyl-, Acetyl-, Chloracetyl-, Trifluoracetyl-, Propionyl-, Butyryl-, Isobutyryl-, $\gamma$-Chlorbutyryl-, Oxalyl-, Succinyl-, Glutamyl-, Pyroglutamyl, Phtalyl -, p-Toluolsulfonyl-Rest ist.

**3.** Chemische Verbindung gemäß Anspruch 1, dadurch gekennzeichnet, daß die durch $R_2$ definierte Schutzgruppe der Aminfunktion vom Typ Urethan, ein tert.-Butyloxycarbonyl-, Isopropyloxycarbonyl-, Ethoxycarbonyl-, Allyloxycarbonyl-, Benzyloxycarbonyl-, Mono- oder Polyhalobenzyloxycarbonyl-, Nitrobenzyloxycarbonyl-Rest ist.

**4.** Verfahren zur Herstellung der Verbindung gemäß Anspruch 1, dadurch gekennzeichnet, daß :
- man die Kupplung eines Peptids der allgemeinen Formel (XIII):

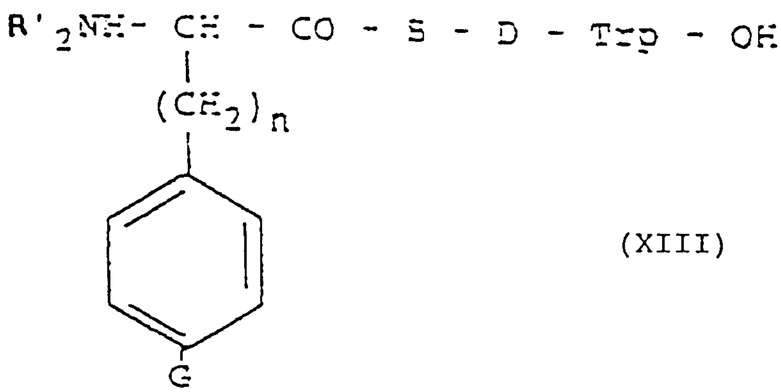

(XIII)

  oder eines aktivierten Derivats dieses Peptids, worin
- $R'_2$ eine Schutzgruppe des Amins wie in Anspruch 1 für $R_2$ definiert ist, darstellt und
- G OH darstellt oder einen Rest der Formel -$(CH_2)_mSO_2OR_3$, wie er bezüglich der Formel (I) definiert ist und
- die anderen Symbole sind die, wie sie im Anspruch 1 in bezug auf die Formel (I) definiert sind, mit einem Tripeptid der Formel (XIV):

  H-E-Asp-$PheNH_2$

  durchführt, worin
- E wie in Anspruch 1 definiert ist, wobei dieses Tripeptid wahlweise geschützt ist,
- dann entfernt man die Schutzgruppe(n) und isoliert das erhaltene Produkt im freien Zustand oder im Salz-Zustand und man überführt es wahlweise in ein anderes Produkt, wie es vorstehend definiert ist, mittels jedes dafür bekannten Verfahrens und man überführt, falls gewünscht, das Endprodukt je nach Fall in ein pharmazeutisch akzeptierbares Salz oder in ein Produkt im freien

Zustand.

5. Verfahren nach Anspruch 4, dadurch gekennzeichnet, daß das Produkt der allgemeinen Formel (XIII) durch Kupplung des Peptids der Formel (XV):

H-B-D-Trp-OH (XV),

in der B und D wie im Anspruch 1 definiert sind, mit einer Aminosäure der allgemeinen Formel (XVI):

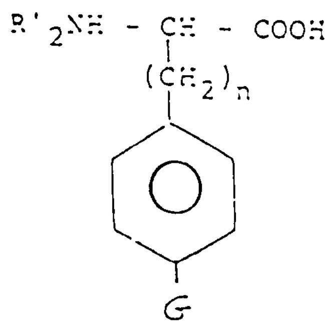

(XVI)

erhalten wird, in welcher:

- G OH darstellt oder einen Rest der Formel $-(CH_2)_m-SO_2OR_3$, wie er in bezug auf Formel (I) definiert ist;
- die Abkürzungen $R'_2$ und n wie vorstehend in bezug auf die Formel (I) definiert sind.

6. Verfahren zur Herstellung der Verbindungen der Formel (I) gemäß Anspruch 1, dadurch gekennzeichnet, daß die Verbindungen durch Kupplung eines Peptids der allgemeinen Formel:

H-B-D-Trp-E-Asp($OR_5$)-Phe-NH-Q (XVII ),

worin

- B, D, E und Q wie im Anspruch 1 definiert sind und
- $R_5$ eine Schutzgruppe aus einer Carbonsäure darstellt und insbesondere einen tert.-Butyl- oder Benzylrest darstellt, mit einer Verbindung der Formel (XVI) wie sie vorstehend definiert ist, erhalten werden.

7. Pharmazeutische Zusammensetzung, dadurch gekennzeichnet, daß sie wenigstens eine gemäß einem der Ansprüche 1 bis 3 definierte Verbindung zusammen mit einem oder mehreren, kompatiblen oder pharmazeutisch akzeptierbaren, Verdünnungsmitteln oder Hilfsstoffen enthält.

8. Zusammensetzung gemäß Anspruch 7, dadurch gekennzeichent, daß sie zur Behandlung nervöser Krankheiten, die vom Zentralnervensystem herstammen, zur Verringerung des Alterns der Nervenzellen, zur Behandlung von Schmerzen bestimmt ist und daß sie als Mittel gegen Anorexie und als Stimulans der Darmtätigkeit angewendet wird.

**Patentansprüche für folgenden Vertragsstaat : GR**

1. Chemische Verbindungen, dargestellt durch die Formel (I):

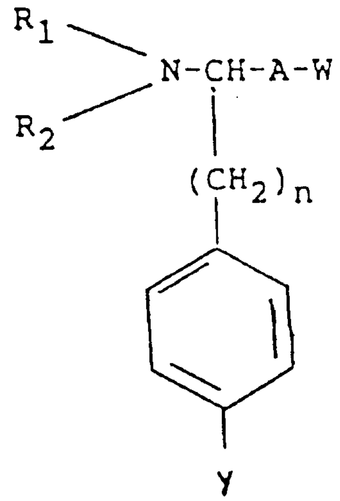

(I)

in der:
- $R_1$ ein Wasserstoffatom darstellt
- $R_2$ eine Schutzgruppe der Aminfunktion vom Typ Acyl oder vom Typ Urethan darstellt
- A -CO darstellt
- Y ein Rest ist, der ausgewählt ist aus solchen der Formel:
- $OSO_2OR_3$ und
- $(CH_2)_m$-$SO_2OR_3$ worin
  * $R_3$ ein Wasserstoffatom oder ein Alkalimetall darstellt;
  * m 1 ist

  n 0 oder 1 ist,

  W : B-D-Trp-E-Asp-PheNHQ darstellt , worin
  * B und E einen Norleucin-Rest oder einen seiner entsprechenden N-methylierten Derivate darstellen,
  * D ein Glycinrest ist,
  * Q Wasserstoff darstellt,

wobei die genannten Verbindungen in den Formen D, L oder DL, vorliegen, ebenso wie deren Mischungen und ihren pharmazeutisch akzeptierbaren Salzen vorliegen.

2. Chemische Verbindung gemäß Anspruch 1, dadurch gekennzeichnet, daß die durch $R_2$ definierte Schutzgruppe der Aminfunktion vom Typ Acyl, ein Formyl-, Acetyl-, Chloracetyl-, Trifluoracetyl-, Propionyl-, Butyryl-, Isobutyryl-, $\gamma$-Chlorbutyryl-, Oxalyl-, Succinyl-, Glutamyl-, Pyroglutamyl, Phtalyl -, p-Toluolsulfonyl-Rest ist.

3. Chemische Verbindung gemäß Anspruch 1, dadurch gekennzeichnet, daß die durch $R_2$ definierte Schutzgruppe der Aminfunktion vom Typ Urethan, ein tert.-Butyloxycarbonyl-, Isopropyloxycarbonyl-, Ethoxycarbonyl-, Allyloxycarbonyl-, Benzyloxycarbonyl-, Mono- oder Polyhalobenzyloxycarbonyl-, Nitrobenzyloxycarbonyl-Rest ist.

4. Verfahren zur Herstellung der Verbindung gemäß Anspruch 1, dadurch gekennzeichnet, daß :

- man die Kupplung eines Peptids der allgemeinen Formel (XIII):

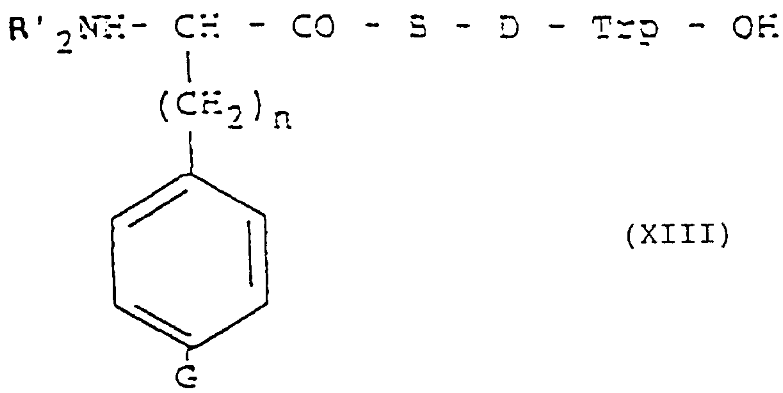

(XIII)

oder eines aktivierten Derivats dieses Peptids, worin
- $R'_2$ eine Schutzgruppe des Amins wie in Anspruch 1 für $R_2$ definiert ist, darstellt und
- G OH darstellt oder einen Rest der Formel $-(CH_2)_mSO_2OR_3$, wie er bezüglich der Formel (I) definiert ist, und
- die anderen Symbole sind die, wie sie im Anspruch 1 in bezug auf die Formel (I) definiert sind, mit einem Tripeptid der Formel (XIV):

H-E-Asp-PheNH$_2$

durchführt, worin
- E wie in Anspruch 1 definiert ist, wobei dieses Tripeptid wahlweise geschützt ist,
- dann entfernt man die Schutzgruppe(n) und isoliert das erhaltene Produkt im freien Zustand oder im Salz-Zustand und man überführt es wahlweise in ein anderes Produkt, wie es vorstehend definiert ist, mittels jedes dafür bekannten Verfahrens und man überführt, falls gewünscht, das Endprodukt je nach Fall in ein pharmazeutisch akzeptierbares Salz oder in ein Produkt im freien Zustand.

**5.** Verfahren nach Anspruch 4, dadurch gekennzeichnet, daß das Produkt der allgemeinen Formel (XIII) durch Kupplung des Peptids der Formel (XV):

H-B-D-Trp-OH (XV),

in der B und D wie im Anspruch 1 definiert sind, mit einer Aminosäure der allgemeinen Formel (XVI):

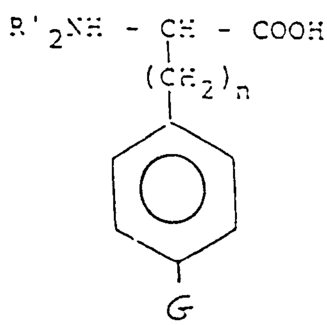

(XVI)

erhalten wird, in welcher:
- G OH darstellt oder einen Rest der Formel $-(CH_2)_m-SO_2OR_3$, wie er in bezug auf Formel (I) definiert ist;
- die Abkürzungen $R'_2$ und n wie vorstehend in bezug auf die Formel (I) definiert sind.

**6.** Verfahren zur Herstellung der Verbindungen der Formel (I) gemäß Anspruch 1, dadurch gekennzeichnet, daß die Verbindungen durch Kupplung eines Peptids der allgemeinen Formel:

H-B-D-Trp-E-Asp($OR_5$)-Phe-NH-Q (XVII ),

worin
- B, D, E und Q wie im Anspruch 1 definiert sind und
- $R_5$ eine Schutzgruppe aus einer Carbonsäure darstellt und insbesondere einen tert.-Butyl- oder Benzylrest darstellt, mit einer Verbindung der Formel (XVI) wie sie vorstehend definiert ist, erhalten werden.

**7.** Verfahren zur Herstellung einer pharmazeutischen Zusammensetzung, dadurch gekennzeichnet, daß man wenigstens eine, gemäß einem der Ansprüche 1 bis 3 definierte Verbindung, mit einem oder mehreren, kompatiblen oder pharmazeutisch akzeptierbaren, Verdünnungsmitteln oder Hilfsstoffen zusammenbringt.

**8.** Verfahren gemäß Anspruch 7, dadurch gekennzeichnet, daß die Zusammensetzung zur Behandlung nervöser Krankheiten, die vom Zentralnervensystem herstammen, zur Verringerung des Alterns der Nervenzellen, zur Behandlung von Schmerzen bestimmt ist und daß sie als Mittel gegen Anorexie und als Stimulans der Darmtätigkeit angewendet wird.

**Patentansprüche für folgenden Vertragsstaat : ES**

**1.** Verfahren zur Herstellung chemischer Verbindungen, dargestellt durch die Formel (I):

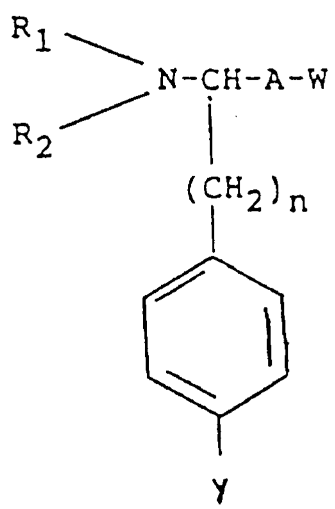

(I)

in der:
- $R_1$ ein Wasserstoffatom darstellt
- $R_2$ eine Schutzgruppe der Aminfunktion vom Typ Acyl oder vom Typ Urethan darstellt
- A -CO darstellt
- Y ein Rest ist, der ausgewählt ist aus solchen der Formel:
- $OSO_2OR_3$ und
- $(CH_2)_m$-$SO_2OR_3$ worin
  - * $R_3$ ein Wasserstoffatom oder ein Alkalimetall darstellt;
  - * m 1 ist

  n 0 oder 1 ist,

  W : B-D-Trp-E-Asp-PheNHQ darstellt , worin
  - * B und E einen Norleucin-Rest oder einen seiner entsprechenden N-methylierten Derivate darstellen,
  - * D ein Glycinrest ist,

* Q Wasserstoff darstellt,

wobei die genannten Verbindungen in den Formen D, L oder DL, vorliegen ebenso wie deren Mischungen und ihren pharmazeutisch akzeptierbaren Salzen , dadurch gekennzeichnet, daß

- man die Kupplung eines Peptids der allgemeinen Formel (XIII):

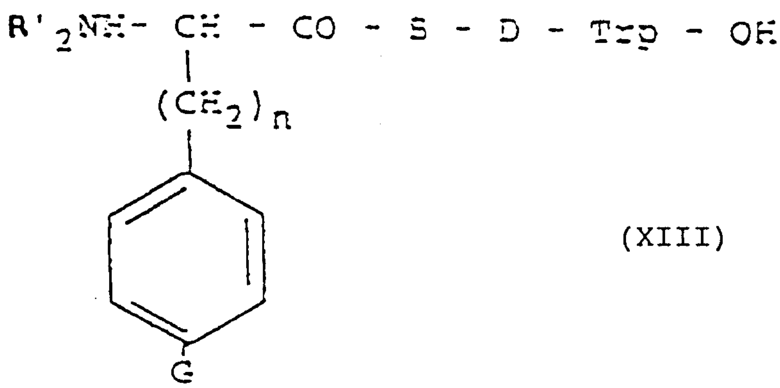

(XIII)

oder eines aktivierten Derivats dieses Peptids, worin

- $R'_2$ eine Schutzgruppe des Amins wie für $R_2$ definiert ist, darstellt und
- G OH darstellt oder einen Rest der Formel $-(CH_2)_mSO_2OR_3$, wie er bezüglich der Formel (I) definiert ist, und
- die anderen Symbole die sind, wie sie in bezug auf die Formel (I) definiert sind,

mit einem Tripeptid der Formel (XIV):

H-E-Asp-PheNH$_2$ (XIV)

durchführt, worin

- E wie vorstehend definiert ist, wobei dieses Tripeptid wahlweise geschützt ist,
- dann entfernt man die Schutzgruppe(n) und isoliert das erhaltene Produkt im freien Zustand oder im Salz-Zustand und man überführt es wahlweise in ein anderes Produkt, wie es vorstehend definiert ist, mittels jedes dafür bekannten Verfahrens und man überführt, falls gewünscht, das Endprodukt je nach Fall in ein pharmazeutisch akzeptierbares Salz oder in ein Produkt im freien Zustand.

**2.** Verfahren nach Anspruch 1, dadurch gekennzeichnet, daß das Produkt der allgemeinen Formel (XIII) durch Kupplung des Peptids der Formel (XV):

H-B-D-Trp-OH

in der B und D wie im Anspruch 1 definiert sind, mit einer Aminosäure der allgemeinen Formel (XVI):

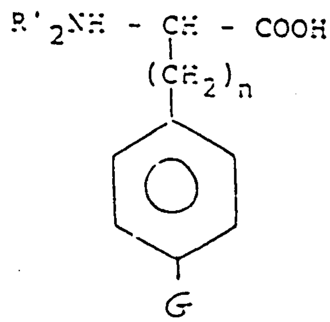

(XVI)

erhalten wird, in welcher:

- G OH darstellt oder einen Rest der Formel $-(CH_2)_m-SO_2OR_3$, wie er in bezug auf Formel (I) definiert ist;

- die Abkürzungen $R'_2$ und n wie vorstehend in bezug auf die Formel (I) definiert sind.

**3.** Verfahren zur Herstellung der Verbindungen der Formel (I) gemäß Anspruch 1, dadurch gekennzeichnet, daß man die Kupplung eines Peptids der allgemeinen Formel:

H-B-D-Trp-E-Asp($OR_5$)-Phe-NH-Q (XVII ),

worin
- B, D, E und Q wie im Anspruch 1 definiert sind und
- $R_5$ eine Schutzgruppe aus einer Carbonsäure darstellt und insbesondere einen tert.-Butyl- oder Benzylrest,

mit einer Verbindung der Formel (XVI) wie sie gemäß Anspruch 2 definiert ist, durchführt.

**4.** Verfahren nach einem der Ansprüche 1 und 3, dadurch gekennzeichnet, daß die durch $R_2$ definierte Schutzgruppe der Aminfunktion vom Typ Acyl, ein Formyl-, Acetyl-, Chloracetyl-, Trifluoracetyl-, Propionyl-, Butyryl-, Isobutyryl-, $\gamma$-Chlorbutyryl-, Oxalyl-, Succinyl-, Glutamyl-, Pyroglutamyl, Phtalyl -, p-Toluolsulfonyl-Rest ist.

**5.** Verfahren nach einem der Ansprüche 1 und 3, dadurch gekennzeichnet, daß die durch $R_2$ definierte Schutzgruppe der Aminfunktion vom Typ Urethan, ein tert.-Butyloxycarbonyl-, Isopropyloxycarbonyl-, Ethoxycarbonyl-, Allyloxycarbonyl-, Benzyloxycarbonyl-, Mono- oder Polyhalobenzyloxycarbonyl-, Nitrobenzyloxycarbonyl-Rest ist.

**6.** Verfahren zur Herstellung einer pharmazeutischen Zusammensetzung, dadurch gekennzeichnet, daß man eine, durch das Verfahren gemäß einem der Ansprüche 1 bis 3 hergestellte, Verbindung mit einem oder mehreren, kompatiblen oder pharmazeutisch akzeptierbaren, Verdünnungsmitteln oder Hilfsstoffen zusammenbringt.

**7.** Verfahren gemäß Anspruch 6, dadurch gekennzeichnet, daß die Zusammensetzung zur Behandlung nervöser Krankheiten, die vom Zentralnervensystem herstammen, zur Verringerung des Alterns von Nervenzellen, zur Behandlung von Schmerzen bestimmt ist und daß sie als Mittel gegen Anorexie und als Stimulans der Darmtätigkeit angewendet wird.